# EUROPEAN PATENT APPLICATION

(11) **EP 4 656 659 A1**
(43) Date of publication of application: **03.12.2025**
(21) Application number: 24178829.8
(22) Date of filing: 29.05.2024
(51) Int. Cl.: C07K 16/46

(54) **MODIFIED FC-ENGAGERS FOR TREATING IMMUNE DISEASES**

(71) Applicant: LoopLab Bio GmbH, 1190 Vienna (AT)
(72) Inventor: Suleiman, Ehsan, 1150 WIEN (AT); Tschismarov, Roland, 1200 WIEN (AT); Stritzker, Jochen, 3400 KIERLING (AT)
(74) Representative: Eisenführ Speiser

(57) **Abstract**

The present invention relates to an scFc-engager comprising asymmetric mutations in its Fc-domains to modulate binding to an immune receptor. Further the invention relates to a vector and a nucleic acid molecule encoding said scFc-engager. The invention also relates to a method of producing said scFc-engager. Further, the invention relates to a pharmaceutical composition comprising said scFc-engager for use in a method of treating and/or preventing a disease.

## Description

### Technical Field

The present invention relates to an scFc-engager comprising asymmetric mutations in its Fc-domains to modulate binding to an immune receptor. Further the invention relates to a vector and a nucleic acid molecule encoding said scFc-engager. The invention also relates to a method of producing said scFc-engager. Further, the invention relates to a pharmaceutical composition comprising said scFc-engager for use in a method of treating and/or preventing a disease.

### Background of the Invention

The immune system's primary function is to distinguish between self and non-self, ensuring protection against pathogens while preventing harm to the body's own tissues. On innate immune effector cells, activating and inhibitory Fc-receptors (FcRs) establish a threshold for activation by immune complexes. Inhibitory FcRs dampen the immune response once an antigen is cleared, preventing excessive inflammation and damage to the host tissues. A well-known example is FcyRIIB, which inhibits B cell receptor signalling and can suppress the activation of other immune cells when engaged. This receptor acts as a checkpoint to balance the effects of activating FcRs, which promote inflammation and immune activation. This critical discrimination involves complex interactions among various immune cells and molecules, including tolerogenic FcRs, which play a vital role in maintaining immune tolerance and preventing autoimmune responses.

In a naturally occurring antibody, the Fc region determines the antibody's isotype and its interactions with FcRs. Each of the five primary antibody isotypes - IgM, IgD, IgG, IgE, and IgA - is defined by its unique Fc-domain. These subtypes exhibit distinctive functional traits, largely due to their varying affinities for FcRs and the distribution of these receptors across different immune cells.

Several human FcRs specific to IgG (FcγRs) have been identified. The immune response involves four activating Fcγ receptors (FcγRI, FcyRIIA, FcγRIIC, and FcγRIIIA) that utilize intracellular tyrosine-based activation motifs, and one inhibitory receptor, FcyRIIB, that uses a tyrosine-based inhibitory motif. FcγRIIIB, found on certain granulocytes, can bind IgG immune complexes potentially without triggering cell activation. Besides FcγRs, which primarily interacting with IgG antibodies, other Fc-domains can bind to a variety of immune receptors, including FcεRI, which specifically binds to IgE antibodies. FcαR is selective for IgA antibodies, while FcRn predominantly interacts with IgG subclasses. For example, CD16a binds to IgG1 and IgG3, which facilitates antibody-dependent cellular cytotoxicity. The C-type Lectin receptor DC-SIGN can recognize carbohydrate structures and may also interact with antibody Fc-domains.

The manipulation of immune signalling pathways for therapeutic purposes is a highly complex endeavour due to the intricate interplay among numerous molecular components. For instance, factors such as the location and shape of the antibody-binding epitope, the stoichiometry of antibody to antigen, and the affinity of the antibody for both its antigen and corresponding Fc-receptors are critical determinants of immune effector cell activation. This activation can lead to a variety of outcomes, including antibody-dependent cellular cytotoxicity (ADCC), antibody-dependent complement deposition (ADCD), antibody-dependent cellular phagocytosis (ADCP), production of neutrophil extracellular traps (NETosis), modulation of T cell responses, antigen immobilization on follicular dendritic cells, plasma cell survival, and platelet activation. Naturally, these processes are vital for the identification and elimination of infected cells, and essential for controlling and eradicating viral infections.

Over the past decades, significant efforts have been made to artificially leverage these complex immune signalling pathways for therapeutic purposes.

However, artificial manipulation harbours the risk of triggering erroneous activation of Fc-receptors, potentially causing uncontrolled immune cell activation and the production of autoantibodies. This misdirected immune response can contribute to various autoimmune diseases-complex disorders where the immune system mistakenly attacks the body's own tissues, leading to severe clinical outcomes if left untreated.

Consequently, there is a critical need for strategies that can target different types of Fc-receptors in a controlled manner. These strategies are necessary both for potential therapeutic interventions aimed at restoring tolerance in autoimmune diseases and for harnessing the immune system to treat a broad spectrum of other diseases.

A key area of focus has been on optimizing the interaction between the Fc-domain and immune receptors, a delicate balance that requires precise tuning of receptor affinity and specificity.

US9394366B2 discloses a CD32B-selective antigen Fc-fusion proteins and monoclonal antibodies (mAbs) designed to link activating receptors with CD32B, aiming, amongst other things, to reduce autoantibodies in autoimmune diseases. Although mutations throughout the Fc-domain of IgG antibodies are claimed, there are several disadvantages that limit their application. Firstly, the Fc-fusion proteins of US9394366B2 are specifically designed to interact solely with the inhibitory Fc-receptor CD32B, ignoring other potentially beneficial inhibitory FcRs, which limits their therapeutic range and effectiveness. Most importantly, the proteins employ a traditional homodimeric and symmetrically engineered Fc-domain, which fails to account for the asymmetric nature of interactions between Fc engagers and receptors, potentially diminishing their functional efficacy in clinical applications. The formation of a dimer comprising two CH2-CH3 units is essential for the functional capabilities of the intact Fc portion of an antibody. This dimerization is supported by interchain disulfide bonds in the hinge region, which maintain the structural integrity of the Fc-molecule.

In cases where the hinge region is absent, the CH3 domains still randomly associate, forming non-covalent dimers. Due to the random pairing of CH3 domains, attaching different binding entities to the CH2-CH3 unit results in a product that is a mix of homodimers and heterodimers, which are challenging to separate biochemically. In addition, the identification and incorporation of relevant mutations within the Fc-domain is labour intensive, involving a finely tuned step-by-step process, as mutations may easily lead to a loss-of-function or a malfunction of the Fc-domain with an undesired immunological outcome.

WO2008131242A1 relates to a single-chain Fc (scFc) polypeptide, which comprises at least two Fc-monomers linked together. These monomers are structured to include a CH2 domain and a CH3 domain that may be connected by a linker sequence. The scFc polypeptide can also incorporate various binding entities that may include different forms of antibody fragments or similar structures. However, WO2008131242A1 discloses a laborious step-by-step procedure for the production of scFc polypeptides, including PCR reactions followed by extensive purification of the amplified DNA and subsequent sequencing. Further, this application does not disclose any method to identify suitable modifications in the Fc-domains that would specifically modulate their receptor binding affinity.

All the aforementioned strategies seek to utilize Fc-domain engagement with specific Fc-receptors to mitigate (auto)immune diseases or cancer. Achieving this level of affinity and specificity for the corresponding immune receptors is an intricate process that starts with identifying suitable mutations in an Fc-domain, followed by the production of Fc-fusion proteins. The subsequent biochemical separation of the target Fc-fusion protein subset from a mixture of other isotypes is, however, very cumbersome. The conventional step-by-step approach, which involves iterative testing along with labour-intensive purification cycles and binding assays, highlights the need for a more efficient, less resource-intensive method for producing Fc-fusion proteins that modulate the immune response.

In particular there is a need to achieve the necessary affinity and specificity for these immune receptors, beginning with the identification of appropriate mutations in an Fc-domain and followed by a streamlined production of Fc-fusion proteins. The traditional method, characterized by iterative testing and labour-intensive cycles of purification and binding assays, underscores the urgent need for a more streamlined, resource-efficient platform approach to produce Fc-fusion proteins that can regulate the immune response. Ideally, this new method would effectively handle a wide range of immune and autoimmune diseases and could be tailored to tackle diseases such as cancer.

Consequently, the current invention seeks to overcome these challenges by introducing a wide range of asymmetrically mutated scFc-engagers, produced through an advanced, less laborious, and more versatile method.

### Summary of the Invention

The above-identified objects are solved by the technical teaching as provided with the present invention.

In a first aspect there is provided an scFc-engager comprising: (i) a first Fc-domain polypeptide comprising or consisting of a hinge-CH2-CH3 domain, (ii) a second Fc-domain polypeptide comprising or consisting of a hinge-CH2-CH3 domain, (iii) optionally: at least one, additional polypeptide comprising at least one immunomodulatory domain and/or at least one targeting domain, wherein the at least one additional polypeptide is positioned at N-terminally and/or the C-terminally at the first and/or the second Fc-domain polypeptide; (iv) and optionally wherein the at least one additional polypeptide and the first and/or second Fc-domain polypeptide respectively, are connected by a linker (v) an interdomain linker connecting the first Fc-domain polypeptide of (i) with the second Fc-domain polypeptide of (ii), and (vi) optionally: a multimerization domain between the at least one additional polypeptide of (iii) and the first Fc-domain polypeptide of (i), and/or optionally, a multimerization domain fused to the C-terminus of the second Fc-domain, wherein the amino acid sequence of the first Fc-domain polypeptide and the amino acid sequence of the second Fc-domain polypeptide differ by at least one functional mutation, wherein the at least one functional mutation is configured to modulate the binding affinity between the combined first and second Fc-domain polypeptide and an immune receptor.

In one embodiment, there is provided an scFc-engager according to the first aspect, wherein the at least one additional polypeptide of (iii) comprises or consists of at least one of a(n) autoantigen, pathogen-associated antigen, cytokine and/or cytokine receptor, immune checkpoint ligand and/or at least one receptor, tumor/cancer-associated (neo)antigen, artificial epitope, imaging/reporter protein, biomarker, enzyme, antibody or variant, or fragment thereof; and optionally at least one immunomodulatory domain and/or at least one targeting domain, or any combination thereof.

In one embodiment there is provided an scFc-engager according to the first aspect or embodiment thereof, wherein the linker of the at least one additional polypeptide and/or the interdomain linker comprise(s) at least one glycan site, preferably a NXT/S motif, and/or at least one stabilizing amino acid mutation and/or at least one binding affinity modulating mutation.

In one embodiment there is provided an scFc-engager according to any one of the first aspects or any embodiments thereof, wherein the first or second scFc-engager domain polypeptide has an amino acid sequence as defined by SEQ ID NOs: 1 to 8, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any one of SEQ ID NOs: 1 to 8.

In a second aspect there is provided at least one nucleic acid molecule encoding an scFc-engager according to the first aspect or any embodiment thereof.

In a third aspect there is provided at least one vector, comprising at least one nucleic acid molecule encoding an scFc-engager according to the first aspect or any embodiment thereof.

In a fourth aspect there is provided at least one self-amplifying biologic, comprising the at least one nucleic acid molecule of the second aspect or at least one vector according to the third aspect.

Another aspect relates to a method of producing an scFc-engager according to the first aspect or any embodiments thereof, the method comprising: (i) introducing at least one nucleic acid molecule according to the second aspect, at least one vector according to the third aspect or at least one self-amplifying biologic according to the fourth aspect into a host cell or in vitro translation system, (ii) culturing or incubating the at least one host cell or the at least one in vitro translation system under conditions suitable for transcription and/or translation of the at least one nucleic acid molecule or the at least one vector, (iii) allowing the assembly of the scFc-engager or the at least one self-amplifying biologic encoded by the at least one nucleic acid molecule or the at least one vector; and (iv) optionally: purifying the scFc-engager or the self-amplifying biologic of step (iii); and (v) obtaining the scFc-engager or the at least one self-amplifying biologic.

One embodiment relates to the method of the previous aspect further comprising a step of: (vi) introducing or contacting the scFc-engager or the at least one self-amplifying biologic, as obtained in step (v) of the previous aspect into/with a target cell or into/with an in vitro cellular system for transfecting said at least one target cell and/or for studying the functionality of the scFc-engager or the at least one self-amplifying biologic.

Another aspect relates to the in vitro use of the scFc-engager according to the first aspect and any embodiment thereof, wherein the scFc-engager is brought into contact with a biological sample.

Another aspect relates to a pharmaceutical composition comprising an scFc-engager according to the first aspect and any embodiment thereof, or a pharmaceutical composition comprising the scFc-engager obtained by or obtainable by a method according the present invention, and optionally, at least one pharmaceutically acceptable carrier or excipient.

Another aspect relates to a pharmaceutical composition comprising at least one self-amplifying biologic, or a pharmaceutical composition comprising the at least one self-amplifying biologic obtained by or obtainable by a method according to the present invention, and optionally, at least one pharmaceutically acceptable carrier or excipient.

In one embodiment the pharmaceutical composition according to the present invention is provided for use in a method of treating and/or preventing a disease, wherein the disease is independently selected from at least one immune disease, autoimmune disease or autoimmune-like condition, at least one inflammatory disease, such as an allergen-, bacterial or viral associated inflammation, a cardiovascular disease and at least one cancer.

In one embodiment a pharmaceutical composition according to the present invention is provided for use in a method of mitigating immunogenicity and/or promoting tolerance induction against at least one biological therapeutic.

One aspect relates to a kit comprising an scFc-engager and/or at least one nucleic acid molecule and/or the at least one vector and/or the at least one self-amplifying biologic according to the present invention, optionally wherein the kit comprises further reagents, including at least one buffer or solution.

Further aspects and embodiments of the present invention can be derived from the subsequent, non-limiting, Detailed Description, the Figures, the Sequence Listing, as well as the attached set of claims.

### Brief Description of Figures

- Figure 1: (FIG 1): Schematic representation of a monomeric, asymmetrically mutated scFc-engager.
- Figure 2: (FIG 2): Schematic representation of a monomeric, asymmetrically mutated scFc-engager with an additional polypeptide fused to at least one immunomodulatory and/or targeting domain as another additional polypeptide.
- Figure 3: (FIG 3): Schematic representation of a multimeric, asymmetrically mutated scFc-engager with application specific protein(s) fused to a scaffolding multimerization domain.
- Figure 4: (FIG 4): Topology of the additional polypeptide and the first (Fc-domain I) and second (Fc-domain II) Fc-domain with minimal mutation space. Specific regions where mutations can be introduced to generate scFc-domains with desired properties are illustrated. The amino acid sequences of wild-type IgG1, IgG2, IgG3, and IgG4 within the relevant region that may be subject to mutation are presented, along with their localization within specific sub-domains of the Fc-domains, e.g., CH2, A-Strand etc. All sequences/regions described adhere to the Eu numbering system.
- Figure 5: (FIG 5): Topology of the additional polypeptide and the first (Fc-domain I) and second (Fc-domain II) Fc-domain with extended mutation space. Specific regions where mutations can be introduced to generate scFc-domains with desired properties are illustrated. The amino acid sequences of wild-type IgG1, IgG2, IgG3, and IgG4 within the relevant region that may be subject to mutation are presented, along with their localization within specific sub-domains of the Fc-domains, e.g., CH2, A-Strand etc. All sequences/regions described adhere to the Eu numbering system.
- Figure 6: (FIG 6): Schematic representation of DC-dependent tolerance induction by tolerogenic Fc-receptors. ITIM: Immunoreceptor Tyrosine-based Inhibitory Motif, ITAM: Immunoreceptor Tyrosine-based Activation Motif
- Figure 7: (FIG 7): Schematic representation of B cell dependent tolerance induction by tolerogenic Fc-receptors. ITIM: Immunoreceptor Tyrosine-based Inhibitory Motif, ITAM: Immunoreceptor Tyrosine-based Activation Motif
- Figure 8: (FIG 8): Schematic representation of DC-dependent immune induction by immunogenic Fc-receptors. ITIM: Immunoreceptor Tyrosine-based Inhibitory Motif, ITAM: Immunoreceptor Tyrosine-based Activation Motif

### Brief Description of Sequences

| **SEQ ID NO:** | **Description** |
|---|---|
| 1 | IgG1 scFc mutation template (truncated hinge-CH1-CH2) protein sequence |
| 2 | IgG2 scFc mutation template (truncated hinge-CH1-CH2) protein sequence |
| 3 | IgG3 scFc mutation template (truncated hinge-CH1-CH2) protein sequence |
| 4 | IgG4 scFc mutation template (truncated hinge-CH1-CH2) protein sequence |
| 5 | IgG1 scFc mutation template (hinge-CH1-CH2) protein sequence |
| 6 | IgG2 scFc mutation template (hinge-CH1-CH2) protein sequence |
| 7 | IgG3 scFc mutation template (hinge-CH1-CH2) protein sequence |
| 8 | IgG4 scFc mutation template (hinge-CH1-CH2) protein sequence |
| 9 | hMOG (1-125) scFc-engager, WT IgG2-based protein sequence |
| 10 | Ovalbumin (OVA) (1-386) scFc-engager, WT IgG2-based protein sequence |
| 11 | human IL-10 scFc-engager, WT IgG2-based protein sequence |
| 12 | human c-Jun bZIP dimerization domain protein sequence, cysteine-flanked |
| 13 | human collagen XVIII trimerization domain protein sequence |
| 14 | full length LC human ferritin (1-175) protein sequence |
| 15 | hMOG (1-125) scFc-engager, WT IgG2-based DNA sequence |
| 16 | OVA (1-386) scFc-engager, WT IgG2-based DNA sequence |
| 17 | human IL-10 scFc-engager, WT IgG2-based DNA sequence |

### Definitions

Unless defined otherwise, technical and scientific terms used herein have the same meaning as commonly understood by one of ordinary skill in the art to which this invention belongs.

An antibody, also known as an immunoglobulin, is a Y-shaped protein mostly made by plasma cells, a type of white blood cell. Antibodies are part of the body's immune system. They recognize and bind to specific foreign substances (antigens), such as bacteria, viruses, or toxins, helping to neutralize or eliminate them. There are five main types of antibodies: IgM, IgG, IgA, IgD, and IgE, each with a distinct function in the immune response.

An autoantigen is a normal protein or molecule in the body that is recognized by the immune system as foreign. This misidentification leads to an immune response against the body's own tissues, resulting in autoimmune diseases. In these conditions, the immune system's antibodies or T cells target and attack cells and tissues that express these autoantigens, causing inflammation, tissue damage, and loss of function in the affected organs.

A biological host comprises every biological entity from a single cell to tissues and organisms wherein foreign material, e.g., an scFc-engager, a vector or a SelfAmp, can be introduced.

A biological sample refers to material derived from a living organism that may be used for analysis, diagnosis, research, and/or therapeutic purposes, such as an organ or part thereof, a tissue, a bodily fluid, a cell, a plurality of cells, or a cell cluster.

A biological therapeutic refers to a type of medication derived from living organisms or their components, such as cells and proteins, used to treat and prevent various diseases. These therapies work by closely mimicking or influencing natural biological processes within the body. Common examples include monoclonal antibodies, vaccines, recombinant proteins, and gene therapies.

Cellular delivery is defined as the process of introducing bioactive substances, such as drugs or genetic material, into cells for therapeutic or research purposes. Methods can be non-viral, including physical techniques like electroporation or chemical ones like lipofection, or viral, using modified viruses as delivery vectors. The objective is to deliver the substance without causing harm and to ensure it reaches its intended cellular site of action.

The CH2 region is one of the constant domains found in the heavy chain of the antibody. It consists of a compact, globular structure and is located proximal to the antigen-binding fragment (Fab) region of the antibody. The CH2 region contains critical binding sites for various effector molecules, such as complement proteins and Fc-receptors present on immune cells. These interactions are essential for initiating effector functions like antibody-dependent cellular cytotoxicity (ADCC), phagocytosis, and complement activation.

The CH3 region is another constant domain found in the heavy chain of the antibody, located distal to the CH2 region towards the C-terminus of the heavy chain. It contributes to the stability of the antibody molecule and provides a platform for inter-chain interactions, particularly in the formation of antibody dimers or higher-order oligomers. The CH3 region also participates in effector functions by interacting with Fc-receptors on immune cells and complement proteins, thereby initiating downstream immune responses.

Combination therapy refers to the use of more than one medication or treatment modality for a single disease or condition. Combination therapy can have advantages of enhanced therapeutic efficacy by targeting multiple aspects of a disease or condition, reducing the likelihood of resistance and/or reducing side effects.

An epitope is the specific part of an antigen that is recognized and bound by an antibody, T cell receptor, or B cell receptor. It is the precise molecular structure within the antigen that interacts with the immune system.

Ex vivo gene therapy is a type of genetic treatment where cells are removed from a patient, genetically modified in a laboratory setting, typically using viral or non-viral vectors to insert a therapeutic gene, and then returned to the patient with the intent to cure or alleviate disease symptoms.

Ex vivo gene transfer refers specifically to the process of introducing new genetic material into the patient's cells while they are outside the body, in a controlled laboratory environment. This transfer is usually achieved through the use of a vector, which carries the desired gene into the cells. Once the cells have been modified, they are infused back into the patient.

A Fab refers to the antigen-binding fragment of an antibody. It is the part of the antibody molecule that specifically binds to an antigen. The Fab region is composed of the variable domains of the heavy and light chains of the antibody and is highly specific to the target antigen.

A functional mutation is defined as a change in a coding sequence that results in a modification of the function of the encoded protein or RNA molecule. This type of mutation alters the biological activity of the encoded molecule, which may affect the organism's phenotype. Functional mutations can either enhance, diminish, or qualitatively change the normal activity of the molecule, leading to various outcomes such as or changes in binding affinity and/or specificity.

The hinge region is a flexible and unstructured region that connects the Fab and Fc-domains in the antibody molecule. It is rich in glycine and proline residues, which confer flexibility to the hinge, allowing it to bend and rotate. The hinge domain provides spatial flexibility to the antibody molecule, allowing the Fab regions to bind antigens at different orientations and distances while maintaining Fc-mediated effector functions. This flexibility is crucial for optimal antigen recognition and engagement, as well as for the accessibility of the Fc-domain to effector molecules. The length and sequence of the hinge domain may vary among different antibody isotypes and subclasses. Further, the hinge region contains cysteine residues that can form disulfide bonds between adjacent antibody heavy chains. These bonds contribute to the stability of the antibody molecule and help maintain its overall structure.

An in vitro cellular system is defined as a controlled experimental setup where cells are studied outside their natural biological context, usually in a petri dish or test tube. In such a system, cells can be derived from various tissues or organs of various organisms, including humans, and are often maintained under specific conditions, e.g., temperature, humidity and/or nutrient supply that mimic their natural environment as closely as possible. The cells can be of a single type (monoculture) or multiple types (co-culture). These systems provide a platform for understanding fundamental biological processes at the cellular level, such as cell growth, division, differentiation, and response to various stimuli e.g., drugs, toxins or hormones, preferably after introduction of the scFc-engager or the self-amplifying biologic into the cells and/or the cell culture.

An inflammatory condition or disease manifests with specific inflammation-related symptoms and the presence of particular biomarkers detectable through medical tests.

Typical symptoms include redness, swelling, heat, and pain at the affected site, alongside systemic indications like fatigue, weight loss, and fever. Blood tests can identify biomarkers indicating inflammation, such as elevated levels of C-Reactive Protein (CRP), an increased Erythrocyte Sedimentation Rate (ESR), elevated white blood cell counts from a Complete Blood Count (CBC), and/or heightened levels of certain cytokines like interleukin-1 (IL-1), interleukin-6 (IL-6), and/or tumor necrosis factor alpha (TNF-α), among others. Additionally, medical imaging tests can detect inflammation in specific organs or tissues. For instance, X-rays may reveal joint damage in rheumatoid arthritis, while colonoscopy or other imaging studies can identify inflammation in the intestines in cases of inflammatory bowel disease.

An immunomodulatory domain is defined as a specific region or sequence within a molecule, typically a protein or peptide that has the ability to modulate the immune system's response, i.e. to either enhance or suppress immune functions. They contain motifs or epitopes that are recognized by immune receptors or other immune-related molecules. Their mechanism of action involves interacting with immune cells, such as T cells, B cells, and dendritic cells, or immune receptors like cytokine receptors and toll-like receptors. This interaction influences signalling pathways that regulate immune responses, including activation, suppression, or modulation of specific immune functions. Co-stimulatory immune domains enhance immune responses by providing necessary signals for immune cell activation, such as CD28 on T cells. Inhibitory domains, like CTLA-4 and PD-1, suppress or down-regulate immune responses to prevent overactivation and autoimmunity. Cytokine domains act as signalling molecules that modulate immune responses by influencing the behaviour of immune cells, including interleukins and interferons. Pattern Recognition Receptor (PRR) domains recognize pathogen-associated molecular patterns (PAMPs) and initiate immune responses, exemplified by toll-like receptor domains. Immunomodulatory domains may be employed for several applications in therapeutics. To example, in cancer immunotherapy, checkpoint inhibitors targeting inhibitory domains like PD-1/PD-L1 enhance anti-tumor immune responses, and Chimeric Antigen Receptor (CAR) T-cell therapy includes co-stimulatory domains to improve T-cell efficacy against cancer cells. For autoimmune diseases, therapies targeting co-stimulatory or inhibitory domains modulate immune responses to reduce autoimmunity. Vaccines may include immunomodulatory domains to enhance the efficacy and specificity of immune responses against pathogens. In transplantation, these domains help modulate immune responses to prevent rejection and improve graft tolerance. The advantages of using immunomodulatory domains include precision, as they allow targeted modulation of specific immune pathways, and versatility, given their application across various diseases including cancer, infectious diseases, and autoimmune disorders. They also facilitate combination therapies to enhance overall efficacy.

A kit is a packaged set of reagents, materials, and optionally instructions, designed to carry out a specific experimental or diagnostic procedure. These kits aim to streamline and simplify the procedure to increase reliability and reproducibility of results.

A pathogen-associated antigen is a specific molecular structure found on the surface of a pathogen, such as a virus, bacterium, or fungus that can be recognized by the immune system. This recognition might trigger an immune response aimed at identifying and neutralizing the pathogen.

A payload is defined as a functional molecule, preferably a nucleic acid molecule or protein molecule that is transported as a cargo to a target cell, where it carries out or influences a biological activity.

A pharmaceutical composition refers to a formulation or mixture prepared for therapeutic or prophylactic use in human or veterinary medicine. It includes an active pharmaceutical ingredient, which provides the intended therapeutic effect, and excipients, which are inactive ingredients that serve roles such as enhancing solubility, improving stability, or aiding in drug delivery.

Recombinant protein expression is a biotechnological process where a specific protein is produced in a host organism that has been genetically modified with a nucleic acid sequence coding for that protein. This technique is used in research, pharmaceutical production, and industrial applications to produce large quantities of specific proteins.

A reporter protein, also referred to as imaging protein, is defined as a protein that is selected for its easily identifiable and measurable characteristics, such as a fluorescent protein, or an enzyme. Changes in the activity or localization of the gene or protein of interest are then indirectly measured via changes in the reporter protein's activity or expression, providing a visible and/or quantifiable readout.

Self-replication is a property of biological systems that allows the construction of an identical or similar copy of itself.

A self-amplifying biologic (Self-Amp) is a biologically-active, synthetic virus-like particle designed for precise delivery to any cell within a mammalian body. It enables targeted cells to produce effectors encoded within the SelfAmp. The immune-evasive outer shell ensures compatibility with a wide range of host organisms and targeted cellular delivery via receptor-binding target moieties. SelfAmps can carry therapeutically active molecules, termed payloads, such as immunomodulatory molecules, thereby aiding in the treatment of various conditions. They can be chemically modified to enhance performance or meet specific needs and may include synthetic molecules. A key advantage is the inherent modular flexibility of SelfAmps. Additionally, components of SelfAmps can be inducible or activatable within the host organism, which allows external control of in vivo expression and replication. Unlike naturally occurring viruses, SelfAmps are engineered for specific biological activities and can harness biomolecular machinery within a host to self-replicate, potentially binding additional molecules from the host to enhance functionality.

A single-chain variable fragment (scFv) is a type of engineered antibody fragment that consists of the variable regions of the heavy (VH) and light chains (VL) of immunoglobulins, connected by a short linker peptide. The linker peptide is flexible and long enough to allow the VH and VL domains to associate and form a binding site that is very similar to that of a full-size antibody. Because they maintain the original antigen specificity and binding affinity of the parent antibody, scFvs are valuable in research, diagnostics, and therapeutics. The smaller size of the scFv compared to a full-length antibody can enhance tissue penetration and accelerate blood clearance. On the other hand, their smaller size can also result in reduced binding avidity due to the loss of the bivalent binding capability of full-size antibodies. To overcome this limitation, scFvs can be engineered into multivalent or multispecific formats.

A targeting domain is defined as a specific region or segment of a molecule, designed to recognize and bind to a particular antigen or receptor on a cell's surface. This domain is engineered to facilitate the selective identification and interaction with specific cells, pathogens, or biomolecules.

A target receptor is defined as a biological molecule that can be bound by a molecule. The binding event triggers a biological signal that influences a cellular pathway or a cellular signalling cascade.

A vector refers to a vehicle or carrier that transfers genetic material from one organism to another. These vectors are typically DNA or RNA molecules such as plasmids, yeast artificial chromosomes, or virus genomes. Vectors are widely used in genetic engineering and biotechnology for various purposes, such as carrying foreign DNA into a host organism where this genetic material can be replicated, transcribed, and translated into protein. Key features of vectors often include a cloning site where the foreign DNA can be inserted, a marker gene to help identify successfully transformed cells, and a promoter sequence to initiate transcription of the inserted gene. For instance, plasmid vectors are small, circular pieces of DNA that can replicate independently of the host's chromosomal DNA. They are often used in bacterial cells for cloning, creating gene libraries, or producing proteins.

Whenever the present disclosure relates to the percentage of identity of nucleic acid or amino acid sequences to each other these values define those values as obtained by using the EMBOSS Water Pairwise Sequence Alignments (nucleotide) programme nucleic acids or the EM-BOSS Water Pairwise Sequence Alignments (protein) programme (https://www.ebi.ac.uk/jdispatcher/psa/emboss_water) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see https://www.ebi.ac.uk/jdispatcher/psa and SMITH, T.F. 5 & WATERMAN, M.S. "Identification of common molecular subsequences" Journal of Molecular Biology, 1981 147 (1):195-197). When conducting an alignment, the default parameters defined by the EMBL-EBI are used.

Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a nucleotide sequence encoding a protein can be "codon optimized" if the respective sequence is to be used in another organism in comparison to the original organism a molecule originates from.

### Detailed Description

Conventional biotechnological therapeutic strategies are trying to address (auto)immune diseases by harnessing the interaction between the Fc-domain and specific Fc-receptors. However, achieving the necessary affinity and specificity for these immune receptors involves a complex process that begins with the identification of appropriate mutations in an Fc-domain, followed by the production of Fc-fusion proteins. A specific kind of Fc-fusion protein is one that fuses the two Fc-domains via a linker, known as single-chain Fc (scFc) fusion proteins. Accordingly, within the present invention, the term "scFc-engager" will be used.

Traditional methods, characterized by iterative testing and labour-intensive cycles of purification and binding assays, underscores the urgent need for a more streamlined, resource-efficient approach to produce Fc-fusion proteins that can regulate the immune response. Ideally, this new method would effectively handle a wide range of immune and autoimmune diseases and could be tailored to tackle elusive diseases such as cancer.

The present invention addresses the complex challenges in Fc-fusion protein production by introducing a novel set of asymmetrically mutated scFc-engagers, produced by an advanced, less laborious, and more versatile method. Central to this innovation is the unique design of the single-chain Fc (scFc) domain of the scFc-engager, which is engineered to enhance binding properties, optimize glycosylation profiles, and facilitate the targeted introduction of mutations. This approach is based on in vitro mutagenesis and yeast display, both aimed at generating and selecting scFc-domains with specific, desirable characteristics.

The essence of the method lies in the creation of mutant libraries, which consist of randomly generated variants that undergo simultaneous mutations within designated areas of the two Fc-domain polypeptides that are linked by a linker, leading to asymmetrical configurations. The linker between the two Fc-domains is an essential component that enables the simultaneous introduction of random mutations into both Fc-domains I and II, which distinguishes the present invention from the prior art. Simultaneous mutagenesis allows a streamlined and accelerated the discovery process. Therefore the present invention unlocks an extensive, unexplored mutagenesis space, which holds the potential to reveal novel Fc variants with unique properties that have eluded traditional methodologies.

In a first aspect there is provided an scFc-engager comprising: (i) a first Fc-domain polypeptide comprising or consisting of a hinge-CH2-CH3 domain, (ii) a second Fc-domain polypeptide comprising or consisting of a hinge-CH2-CH3 domain, (iii) optionally: at least one, additional polypeptide comprising at least one immunomodulatory domain and/or at least one targeting domain, wherein the at least one additional polypeptide is positioned at N-terminally and/or the C-terminally at the first and/or the second Fc-domain polypeptide; (iv) and optionally wherein the at least one additional polypeptide and the first and/or second Fc-domain polypeptide respectively, are connected by a linker (v) an interdomain linker connecting the first Fc-domain polypeptide of (i) with the second Fc-domain polypeptide of (ii), and (vi) optionally: a multimerization domain between the at least one additional polypeptide of (iii) and the first Fc-domain polypeptide of (i), and/or optionally, a multimerization domain fused to the C-terminus of the second Fc-domain, wherein the amino acid sequence of the first Fc-domain polypeptide and the amino acid sequence of the second Fc-domain polypeptide differ by at least one functional mutation, wherein the at least one functional mutation is configured to modulate the binding affinity between the combined first and second Fc-domain polypeptide and an immune receptor.

The single-chain Fc (scFc) format offers several advantages over traditional antibody formats. Especially, the simplification in manufacturing can translate to cost savings and increased scalability, making scFc-molecules more accessible for large-scale production. Furthermore, the single-chain nature of scFc antibodies allows for precise engineering of the linker between the two Fc-domains. This precision enables the simultaneous introduction of mutations and provides an opportunity to incorporate property-modifying residues.

In certain embodiments, the sequence identity between the first and the second Fc-domain is below 85%; more preferably below 65%, wherein the length of identical sequence stretches may be below 40 base pairs, more preferably below 25 base pairs.

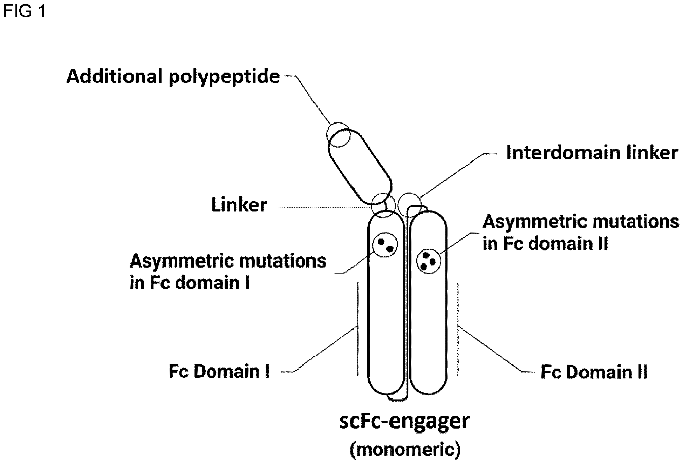

In one embodiment, the scFc-engager is arranged in the following order from the N-terminus to the C-terminus: the at least one additional polypeptide, the first Fc-domain, the interdomain linker, and the second Fc-domain. This arrangement is schematically illustrated in FIG 1.

In one embodiment, the scFc-engager is arranged in the following order from N- to C-terminus: the first Fc-domain, optionally the interdomain linker or a fragment thereof, at least one additional polypeptide, optionally the interdomain linker or a fragment thereof, and the second Fc-domain.

In one embodiment, the scFc-engager is arranged in the following order from the N-terminus to the C-terminus: the first Fc-domain, the interdomain linker, the second Fc-domain, and the at least one additional polypeptide.

In one embodiment, the scFc-engager is arranged in the following order from the N-terminus to the C-terminus: the at least one additional polypeptide, the first Fc-domain, the interdomain linker, the second Fc-domain, the at least one additional polypeptide.

In certain embodiments the at least one additional polypeptide comprises DSG1, DSG3, BP230, BP180, COL17A1, COL7, gluten peptides, such as those presented by HLA-DQ2 and HLA-DQ8, tTG, TG2, GPllb, GPllla, GP-Ib-IX-V, TPO-RAs (thrombopoietin receptor agonists), ADAMTS13, proinsulin, insulin B chain 9-23, COL2A1, GPI, MBP, PLP, AQP4, MOG, mimotopes of dsDNA, a nucleosomal histone, mimotopes of an snRNP, ASGPR, MYH6, PDC-E2, AChR, MuSK, parietal cell components, such as H+/K+ ATPase, viral antigens, such as HIV-1, HCV and EBV, bacterial antigens, such as those originating from streptococcus pneumoniae, mycobacterium tuberculosis, bacillus anthracis, tumor-associated antigens, such as MAGE-A3, HER2, MUC1 and CD20, neoantigens, non-modified APOB peptides, Hsp60, GRP78, ApoA1, β2GP1, cardiolipin, oxidation-specific epitopes, MDA-modified APOB peptides, p45, p210, PC, pCETP, ALDH4A1, ADAMST7, mimotopes of capsular polysaccharides of streptococcus pneumoniae, epitopes against which B1 cells have specificity, MerTK receptor agonists and ligands, such as Gas6 and Protein S, MFG-E8, IL-2, IL-10, TGF-β, INF-α, TNFR, IL1R, VEGFR, PD-L1, CD40L, ICOSL, CD80, CD86, BAFF, PD-1, CD40, ICOS, CTLA4, CD28, BAFFR, EPO and GLP-1, but not limited thereof.

In certain embodiments, the at least one additional polypeptide may be functionalized for linking at least one non-peptide molecule, such as a polysaccharide, a carbohydrate, a nucleic acid and/or a lipid thereto, wherein the nucleic acid may comprise at least one chemical modification of the nucleic acid sugar moiety, phosphate backbone or nucleobase.

To establish a chemical linkage between the at least one additional polypeptide and at least one non-peptide molecule, any method known in the art may be used, comprising chemical conjugation, such as amide bond formation, thiol-maleimide reaction or click chemistry, enzymatic conjugation, such as sortase-mediated or transglutaminase-mediated conjugation.

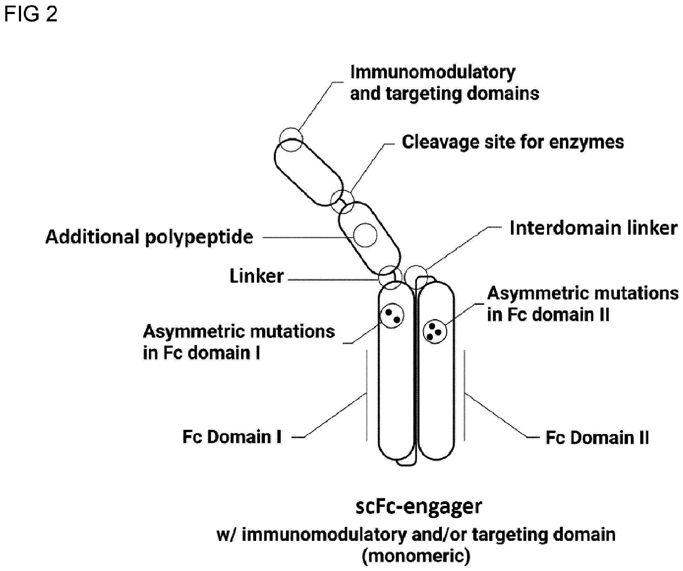

In certain embodiments one, or more than one, preferably two, additional polypeptides may be sequentially connected to each other through a linker, respectively. This arrangement is schematically illustrated in FIG 2.

Preferably, the order of the sequentially connected more than one additional polypeptides depends on the therapeutic application.

In certain embodiments, a linker may include a cleavage site for any protease known in the art.

In certain embodiments, a linker may include a cleavage site for enzymes from classes such as proprotein convertases (PCs), matrix metalloproteinases (MMPs), a disintegrin and metalloproteinase (ADAM), a disintegrin and metalloproteinase with thrombospondin motifs (ADAMTS) or hepatocyte growth factor activator (HGFA), but not limited thereof.

The multimerization of scFc-engagers enhances their avidity and modifies their binding behaviour to immune receptors, i.e., multimerization increases the local concentration of Fc regions, enabling stronger and more simultaneous interactions with multiple binding sites on immune receptors. Consequently, a higher avidity might have an amplifying effect on immune signalling by scFc-engagers.

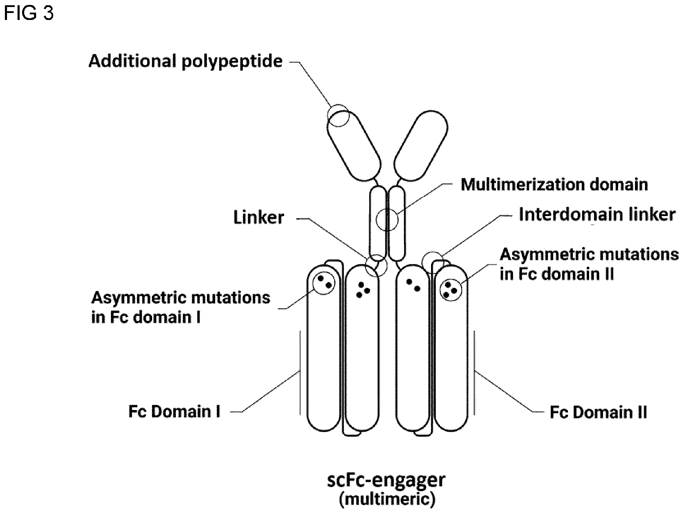

One embodiment of a multimerized scFc-engager is schematically illustrated in FIG 3.

In certain embodiments the multimerization domain may be positioned at the N- or C-terminus of the first and/or second Fc-domain and/or between the first and/or Fc-domains and the at least one additional polypeptide.

Preferably, the multimerization domain is selected from the group consisting of a human c-Jun bZIP dimerization domain (i) without the basic domain, (ii) with LERIAR spacer or (iii) cysteine-flanked, a collagen trimerization domain and a protein-based nanoparticle assembly, such as ferritin-based nanoparticles, or any multimerization domain known in the art, but not limited thereof.

In certain embodiments, the multimerization domain may be a cysteine-flanked c-Jun bZIP dimerization domain, wherein the cysteine-flanked c-Jun bZIP dimerization domain has an amino acid sequence as defined by SEQ ID NO: 12, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any one of SEQ ID NO: 12.

In certain embodiments, the multimerization domain may be a human collagen XVIII trimerization domain, wherein the a human collagen XVIII trimerization domain has an amino acid sequence as defined by SEQ ID NO: 13, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any one of SEQ ID NO: 13.

In certain embodiments, the multimerization domain may be LC human ferritin, wherein the LC human ferritin has an amino acid sequence as defined by SEQ ID NO: 14, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any one of SEQ ID NO: 14.

Immune receptors exhibit a certain degree of structural asymmetry, which significantly influences their binding affinity and specificity towards Fc-domains. This asymmetry can result from variations in the spatial arrangement of receptor subunits or differing affinities of these subunits for the Fc region. As a result, when an immune receptor engages with an Fc-domain, the asymmetric arrangement can enhance the interaction's selectivity and strength. The unique configuration allows these receptors to bind more precisely to specific sites on the Fc-domain, which facilitates a more targeted and effective immune response. This precise alignment not only improves the overall stability of the Fc-domain-receptor complex but also ensures that signalling pathways specific to the immune response are accurately triggered, which enhances downstream immune signalling.

In certain embodiments, the immune receptor may be a non-canonical (Type II) FcR, whereas the non-canonical (Type II) Fc-receptor (FcR) may be a Clec (C-type lectin), such as CD206 (Mannose receptor) or CD209 (DC-SIGN), but not limited thereof.

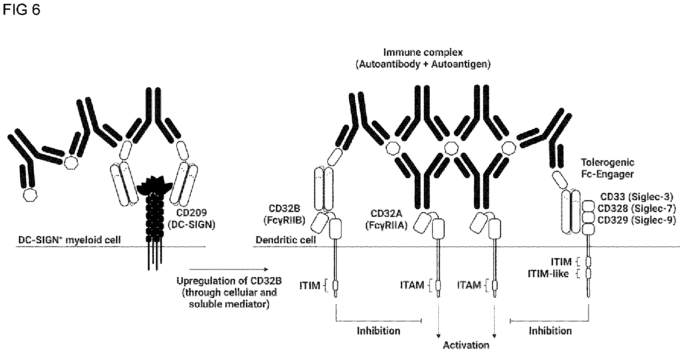

The engagement of scFc-engagers with CD209 (DC-SIGN) is schematically illustrated in FIG. 6.

In certain embodiments, the immune receptor may be a non-canonical (Type II) FcR, whereas the non-canonical (Type II) Fc-receptor (FcR) may be a Siglec (Sialic acid-binding immunoglobulin-type lectin), such as CD22 (Siglec-2), CD23 (FcεRII), CD33 (Siglec-3), CD328 (Siglec-7), CD329 (Siglec-9) or Siglec-1 0, but not limited thereof.

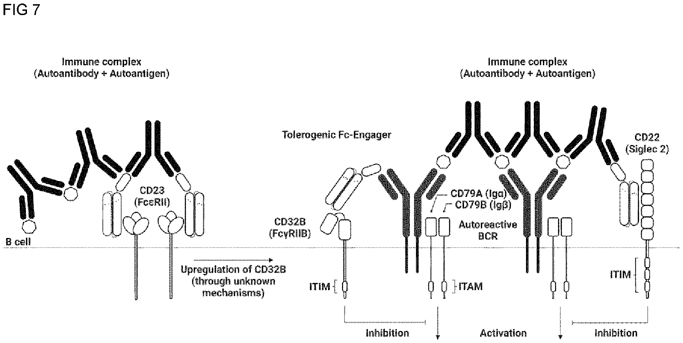

The engagement of scFc-engagers with CD23 (FcεRII) is schematically illustrated in FIG. 7.

In certain embodiments, the immune receptor may be a canonical (Type I) Fc-receptor (FcR), whereas the canonical (Type I) Fc-receptor (FcR) may be an activating FcγR, such as CD64 (FcγRI), CD32A (FcγRIIA), CD16A (FcγRIIIA) or CD16B (FcγRIIIB) or an inhibitory FcγR, such as CD32B (FcγRIIB), but not limited thereof.

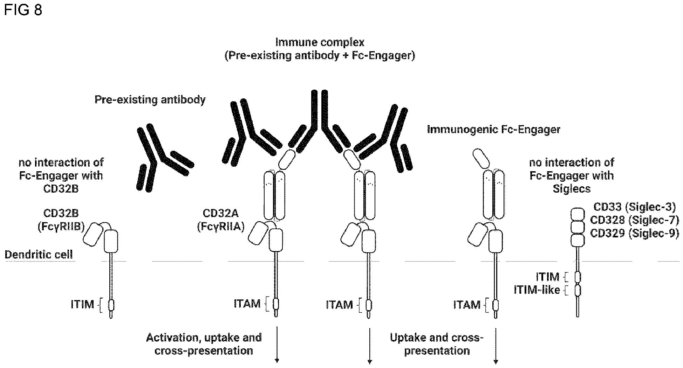

The engagement of scFc-engagers with CD32B (FcγRIIB) is schematically illustrated in FIG. 8.

In certain embodiments, the immune receptor may be FcRn (neonatal Fc-receptor), C1q (Complement component 1q) or CD305 (LAIR1; Leukocyte-associated immunoglobulin-like receptor 1), but not limited thereof.

In one embodiment, there is provided an scFc-engager, wherein the at least one additional polypeptide of (iii) comprises or consists of at least one of an autoantigen, pathogen-associated antigen, cytokine and/or cytokine receptor, immune checkpoint ligand and/or at least one receptor, tumor/cancer-associated (neo)antigen, artificial epitope, imaging/reporter protein, biomarker, enzyme, antibody or variant, or fragment thereof; and optionally at least one immunomodulatory domain and/or at least one targeting domain, or any combination thereof.

In certain embodiments, the at least one additional polypeptide gains additional functionality by at least one chemical modification, comprising, but not restricted to, a chemical modification of a canonical amino acid, such as a modification of a lysine, the protein N-terminal primary amine, a cysteine residue, an aromatic residue, or the incorporation of a non-canonical amino acid via e.g., genetic code expansion, site-specific amino acid incorporation or residue-specific amino acid incorporation.

In certain embodiments, the imaging/reporter protein comprises at least one fluorescent protein, such as Green Fluorescent Protein (GFP), Cyan Fluorescent Protein (CFP), Yellow Fluorescent Protein (YFP) or Red Fluorescent Protein (RFP), luciferases, such as firefly luciferase or Renilla luciferase, beta-galactosidase, alkaline phosphatase, horseradish peroxidase (HRP), chloramphenicol acetyltransferase (CAT) or aequorin, but not limited thereto.

In certain embodiments, the imaging/reporter protein enables optical imaging, magnetic resonance imaging, PET-imaging, SPECT-imaging, ultrasound-imaging, and/or optoacoustic imaging, but not limited thereto.

In one embodiment, the at least one fluorescent protein has a near-infrared excitation and emission spectrum.

In one embodiment there is provided an scFc-engager, wherein the linker of the at least one additional polypeptide and/or the interdomain linker comprise(s) at least one glycan site, preferably a NXT/S motif, and/or at least one stabilizing amino acid mutation and/or at least one binding affinity modulating mutation.

In certain embodiments, the linker of the at least one additional polynucleotide may be any sufficiently flexible linker known in the art.

In certain embodiments, the linker may be a (GGGGS)ₙ linker, whereas the (GGGGS)ₙ linker optionally comprises at least on stabilizing mutation, at least one binding-modulating mutation and/or at least one NXT/S N-Glycan motif, wherein n is any natural number representing a linker length known in the art.

In certain embodiments, the linker of the at least one additional polynucleotide may be a (GGGGS)ₙ linker, whereas the (GGGGS)ₙ linker optionally comprises at least one stabilizing mutation, at least one binding-modulating mutation and/or at least one NXT/S N-Glycan motif, wherein n is in the range of 1 to 20, preferably in the range of 1 to 10, most preferably in the range to 2 to 6.

In certain embodiments, the interdomain linker may be a (GGGGS)ₙ linker, whereas the (GGGGS)ₙ linker optionally comprises at least one stabilizing mutation, at least one binding-modulating mutation and/or at least one NXT/S N-Glycan motif, wherein n is in the range of 1 to 50, preferably in the range of 2 to 25, most preferably in the range of 8 to 12.

In one embodiment there is provided an scFc-engager, wherein the first or second scFc-engager domain polypeptide has an amino acid sequence as defined by SEQ ID NOs: 1 to 8, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any one of SEQ ID NOs: 1 to 8.

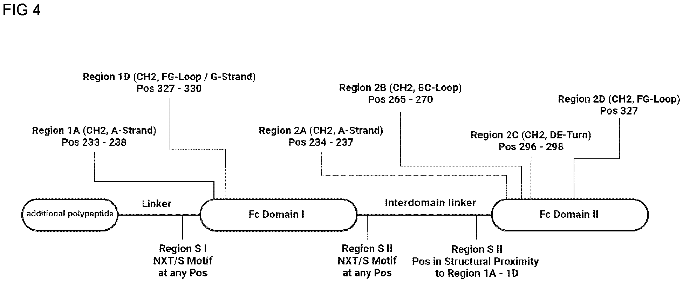

A preferred minimal mutation space of the first and second Fc-domains is schematically illustrated in FIG 4.

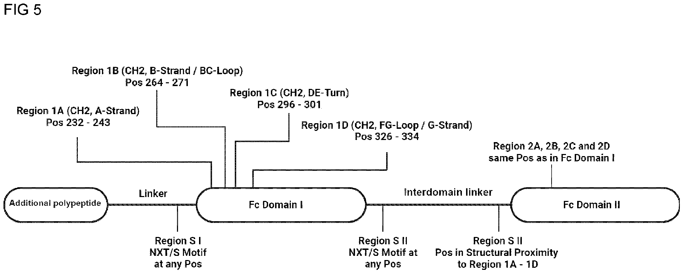

A preferred extended mutation space of the first and second Fc-domains is schematically illustrated in FIG 5.

In a second aspect there is provided at least one nucleic acid molecule encoding an scFc-engager.

In a third aspect there is provided at least one vector comprising at least one nucleic acid molecule encoding an scFc-engager.

The person skilled in the art is aware of several techniques to clone a nucleic acid and functionally insert a nucleic acid sequence encoding a protein of interest into a vector or to mutate the nucleic acid sequence. In this context, a functional insertion means that the introduced sequence allows for the transcription and translation of all nucleic acid sequences encoded by the vector. This requires that the insertion does not disrupt any regulatory sequences, such as promoters, nor compromise the structural and functional integrity of proteins encoded by the nucleic acid sequence.

In certain embodiments, the at least one vector may be delivered to a cell by transport through a carrier, comprising a nanoparticle, such as a lipid nanoparticle (LNP), a sold lipid nanoparticle (SLN) or a polymeric nanoparticle, a dendrimer, a viral or virus-like capsid, an exosome, a liposome, a micelle, but not limited thereto.

In another aspect there is provided at least one self-amplifying biologic, comprising the at least one nucleic acid molecule or at least one vector.

In a further embodiment, there is provided a kit comprising the at least one self-amplifying biologic, and/or the at least one vector encoding the at least one self-amplifying biologic, optionally wherein the kit comprises further reagents, including at least one buffer or solution.

The kit will usually contain all ingredients, with or without suitable expression host cells that allow the amplification and/or assembly of a functional self-amplifying biologic.

Another aspect relates to a method of producing an scFc-engager, the method comprising: (i) introducing at least one nucleic acid molecule, at least one vector or at least one self-amplifying biologic into a host cell or in vitro translation system, (ii) culturing or incubating the at least one host cell or the at least one in vitro translation system under conditions suitable for transcription and/or translation of the at least one nucleic acid molecule or the at least one vector, (iii) allowing the assembly of the scFc-engager or the at least one self-amplifying biologic encoded by the at least one nucleic acid molecule or the at least one vector; and (iv) optionally: purifying the scFc-engager or the self-amplifying biologic of step (iii); and (v) obtaining the scFc-engager or the at least one self-amplifying biologic.

One embodiment relates to the method of the previous aspect further comprising a step of: (vi) introducing or contacting the scFc-engager or the at least one self-amplifying biologic, as obtained in step (v) of the previous aspect into/with a target cell or into/with an in vitro cellular system for transfecting said at least one target cell and/or for studying the functionality of the scFc-engager or the at least one self-amplifying biologic.

Another aspect relates to the in vitro use of the scFc-engager, wherein the scFc-engager is brought into contact with a biological sample.

In certain embodiments, the scFc-engager may be brought into contact with a biological sample to assess binding affinity and/or specificity towards an immune receptor.

In certain embodiments, the scFc-engager may be brought into contact with a biological sample to conduct a functional assay, evaluating its ability to inhibit or stimulate a cellular function, such as modulating immune signalling.

In certain embodiments, the scFc-engager may be brought into contact with a biological sample to perform cytotoxicity tests, including antibody-dependent cellular cytotoxicity (ADCC) and complement-dependent cytotoxicity (CDC) assays.

In certain embodiments, the scFc-engager may be brought into contact with a biological sample to conduct internalization and trafficking studies.

In certain embodiments, the scFc-engager may be brought into contact with a biological sample to examine off-target effects and/or to identifying any unintended interactions with the biological sample.

In certain embodiments, the scFc-engager may be brought into contact with a biological sample to establish a dose-response relationship.

In certain embodiments, the scFc-engager may be used as a carrier protein and/or a half-life extender.

In one embodiment the scFc-engager may be used as a carrier protein by attaching a therapeutic agents, such as a drug, a toxin, or a radioactive substance, to it, to achieve cellular delivering of said therapeutic agent.

In one embodiment the scFc-engager may be used as a half-life extender by attaching a therapeutic agent, such as a drug, a toxin, or a radioactive substance, to it, wherein the scFc-engager may be mutated aiming for increased affinity to the FcRn receptor.

Another aspect relates to a pharmaceutical composition comprising an scFc-engager, or a pharmaceutical composition comprising the scFc-engager obtained by or obtainable by a method according the present invention, and optionally, at least one pharmaceutically acceptable carrier or excipient.

In certain embodiments, the pharmaceutically acceptable carrier or excipient comprises at least one bulking agent and/or at least one binder and/or at least one disintegrant and/or at least one coating and/or at least one filler or diluent and/or at least one stabilizer or preservative and/or at least one solvent and/or at least one solubilizer.

In certain embodiments, said pharmaceutical composition is used as part of a combination therapy, wherein the pharmaceutical composition can be used in combination with any medicament known in the art, such as small molecules or biologics, or with treatment modalities known in the art, such as antibody therapy, chemotherapy, radiation therapy, immunotherapy, hormone therapy or gene therapy, but not limited thereto.

In certain embodiments, the pharmaceutical composition may be used as part of a combination therapy, wherein combination therapy involves the concurrent use of multiple medications or treatment modalities for use in a method of treating and/or preventing a disease or condition.

In certain embodiments, the pharmaceutical composition may be combined with known medications, such as steroids, and biologics for use in treating and/or preventing a disease or condition.

Another aspect relates to a pharmaceutical composition comprising at least one self-amplifying biologic, or a pharmaceutical composition comprising the at least one self-amplifying biologic obtained by or obtainable by a method according to the present invention, and optionally, at least one pharmaceutically acceptable carrier or excipient.

In one embodiment the pharmaceutical composition according to the present invention is provided for use in a method of treating and/or preventing a disease, wherein the disease is independently selected from at least one immune disease, autoimmune disease or autoimmune-like condition, at least one inflammatory disease, such as an allergen-, bacterial or viral associated inflammation, a cardiovascular disease and at least one cancer.

Treating and/or preventing a disease comprises mitigating a disease, wherein mitigating focuses on reducing its severity and the associated symptoms in individuals who are already affected. This can involve symptom management strategies, supportive care, and interventions that minimize complications, thus improving the quality of life and outcomes for patients.

In certain embodiments the pharmaceutical composition according to the present invention is provided for use in a method of administering a prophylactic and/or a therapeutic vaccination against a viral and/or bacterial infection.

In certain embodiments, the pharmaceutical composition is provided for use in a method of treating or preventing an adverse immune reaction in enzyme replacement therapy, gene therapy, in organ or tissue transplantation, and/or in xenotransplantation.

In certain embodiments, the pharmaceutical composition is provided for use in a method of treating or preventing an immune reaction in a patient with hemophilia undergoing enzyme replacement therapy with recombinant FVIII and/or FIX, wherein the pharmaceutical composition comprises a tolerogenic scFc fused to FVIII or FIX.

In certain embodiments the pharmaceutical composition is provided for use in a method of treating and/or preventing a disease, wherein the disease is an autoimmune disease or autoimmune-like condition comprising skin blistering diseases, pemphigus vulgaris, bullous pemphigoid, epidermolysis bullosa acquisita, celiac disease, thrombocytopenic purpura, immune thrombocytopenia, thrombotic thrombocytopenic purpura, Type 1 Diabetes, rheumatoid arthritis, demyelinating autoimmune disorders, multiple sclerosis, neuromyelitis optica, MOG antibody disease, systemic lupus erythematosus, autoimmune hepatitis, autoimmune myocarditis, primary biliary cholangitis, myasthenia gravis, autoimmune gastritis, granulomatosis with polyangiitis (GPA), Membranous glomerulonephritis (MGN), Autoimmune Addison's Disease (AAD), Autoimmune Autonomic Ganglionopathy (AAG), Small Fiber Sensory Neuropathy (SFSN), small fiber inflammatory bowl disease, Crohn's disease, ulcerative colitis, undifferentiated connective tissue disease, acromegaly, acquired aplastic anemia, Agammaglobulinemia, primary immunodeficiency, also known as XLA or ARA, Alopecia areata, Antiphospholipid syndrome (APS), Autoimmune Polyglandular Syndromes (APS), Grave's disease, Autoimmune Hemolytic Anemia (AIHA), testicular autoimmunity, ocular cicatricial pemphigoid, neuromyelitis optica, also known as Devic's disease, Immune thrombocytopenia (ITP), also known as autoimmune thrombocytopenia purpura, IgA nephropathy, also known as Berger's disease, Hurst's disease, also known as Acute hemorrhagic leukoencephalitis (AHLE), Autoimmune encephalitis (AE), Acute disseminated encephalomyelitis (ADEM), Pure red cell aplasia (PRCA), Myasthenia gravis (MG), Mixed connective tissue disease (MCTD), Ménière's disease, Lupus nephritis (LN), Linear IgA disease (LAD), Lambert-Eaton myasthenic syndrome (LEMS), Henoch-Schönlein purpura (HSP)/lgA vasculitis, Hashimoto's thyroiditis, also known as autoimmune thyroiditis, Guillain-Barrè syndrome (GBS), Goodpasture's syndrome/Anti-GBM/Anti-TBM disease, Glomerulonephritis (GN), Evans syndrome, eosinophilic fasciitis, Balo disease, also known as concentric sclerosis, Behçet's disease, Bullous pemphigoid, Discoid lupus erythematosus (DLE), dermatomyositis, Dermatitis Herpetiformis (DH), Cold Agglutinin Disease (CAD), Churg-Strauss syndrome, also known as Eosinophilic Granulomatosis with Polyangiitis (EGPA), Chronic Inflammatory Demyelinating Polyneuropathy (CIDP), autoimmune gastritis, autoimmune myocarditis, autoimmune oophoritis, Autoimmune Pancreatitis (AlP), Autoimmune progesterone dermatitis (APD), palindromic rheumatism, Paraneoplastic Cerebellar Degeneration (PCD), multiple sclerosis, celiac disease, acquired hemophilia, Microscopic Polyangiitis (MPA), also known as ANCA-associated vasculitis, neutropenia, Rasmussen's encephalitis, Stiff Person Syndrome (SPS), thrombotic thrombocytopenic purpura, narcolepsy, pemphigus vulgaris, pemphigus foliaceus, paraneoplastic pemphigus, epidermolysis bullosa acquisita, cicatricial pemphigoid, Pemphigoid Gestationis (PG), autoimmune blistering disorders, Paroxysmal Nocturnal Hemoglobinuria (PNH), Parsonage-Turner syndrome (PTS), POEMS syndrome, Polyarteritis Nodosa (PAN), polymyalgia rheumatica, polymyositis, pernicious anemia, uveitis, vitiligo, primary biliary cholangitis (PBC), Palmoplantar Pustulosis (PPP), inflammatory idiopathic myopathies and/or Autoimmune Inner Ear Disease (AIED), but not limited thereof.

In certain embodiments the pharmaceutical composition is provided for use in a method of treating and/or preventing a disease, wherein the strategy for treating and/or preventing comprises tolerance induction in the context of atherosclerosis, active immunization in the context of atherosclerosis and stimulation of efferocytosis.

Preferably, the choice of selecting the at least one additional polypeptide according the present invention depends on the therapeutic indication or application.

Preferably, for preventing and/or treating an autoimmune disease, the at least one additional polypeptide may be selected from the group as defined in **Table 1,** but not limited thereof, dependent on the respective indication or application, or may be any scFv, Fab and/or fragment of the additional polypeptide as defined in **Table 1.**

**Table 1**

| **Indication / Application** | **Additional polypeptide** |
|---|---|
| Pemphigus vulgaris | DSG1, DSG3 |
| Bullous pemphigoid | BP230, BP180 (COL17A1) |
| Epidermolysis bullosa acquisita | COL7 |
| Celiac disease | Gluten peptide (specifically those presented by HLA-DQ2 and HLA-DQ8), tTG / TG2 |
| Immune thrombocytopenia | GPIlb/Illa and GP-Ib-IX-V, TPO-RAs (thrombopoietin receptor agonists) |
| Thrombotic thrombocytopenic purpura | ADAMTS13 |
| Type 1 Diabetes | Proinsulin, insulin B chain 9-23 |
| Rheumatoid arthritis | COL2A1, GPI |
| Multiple sclerosis | MBP, PLP |
| Neuromyelitis optica | AQP4 |
| MOG antibody disease | MOG |
| Systemic lupus erythematosus | dsDNA, nucleosomal histones, snRNPs |
| Autoimmune hepatitis | ASGPR |
| Autoimmune myocarditis | MYH6 |
| Primary biliary cholangitis | PDC-E2 |
| Myasthenia gravis | AChR, MuSK |
| Autoimmune gastritis | Parietal cell components, e.g., H+/K+ ATPase |
| Granulomatosis with polyangiitis (GPA) | Proteinase 3 (PR3), myeloperoxidase (MPO) |
| Membranous glomerulonephritis (MGN) | Phospholipase A2 Receptor (PLA2R) |

Preferably, for the mitigating, preventing and/or treating of hemophilia, the at least one additional polypeptide may be FVIII and/or FIX.

In certain embodiments, the disease may be a cancer, such as bladder cancer, breast cancer, colon cancer, rectal cancer, endometrial cancer, kidney cancer, leukemia, liver cancer, lung cancer, melanoma, non-Hodgkin lymphoma, pancreatic cancer, prostate cancer or thyroid cancer, but not limited thereof. A more comprehensive list of eligible types of cancer is published by the NIH National Cancer Institute (https://www.cancer.gov/types).

Preferably, for the use in prophylactic and therapeutic vaccination, the at least additional polypeptide may be selected from the group as defined in **Table 2,** but not limited thereof, dependent on the respective indication or application, or may be any scFv, Fab and/or fragment of the additional polypeptide as defined in **Table 2.**

**Table 2**

| **Indication / Application** | **Additional polypeptide** |
|---|---|
| Viruses | Viral antigens of e.g., HIV-1, HCV, EBV |
| Bacteria | Bacterial antigens of e.g., streptococcus pneumoniae, mycobacterium tuberculosis, bacillus anthracis. |
| General (off-the-shelf) | Tumor-associated antigens, e.g., MAGE-A3, HER2, MUC1, CD20 |
| Personalized medicine | Neoantigens. |

Preferably, for the use in the method of resolving a chronic inflammatory disease, the at least one additional polypeptide may be selected from the group as defined in **Table 3,** but not limited thereof, dependent on the respective indication or application, or may be any scFv, Fab and/or fragment of the additional polypeptide as defined in **Table 3.**

**Table 3**

| **Indication / Application** | **Additional polypeptide** |
|---|---|
| Tolerance induction in the context of atherosclerosis | non-modified APOB peptides, Hsp60, GRP78, ApoA1, β2GP1, cardiolipin |
| Active immunization in the context of atherosclerosis | oxidation-specific epitopes, MDA-modified APOB peptides, p45, p210, PC, pCETP, ALDH4A1, ADAMST7, mimotopes of capsular polysaccharides of streptococcus pneumoniae and other epitopes against which B1 cells have specificity |
| Stimulation of efferocytosis | MerTK receptor agonists / ligand (e.g., Gas6, Protein S), MFG-E8 |

Preferably, for the use in immunomodulation, the additional polypeptide may be selected from the group as defined in **Table 4**, but not limited thereof, dependent on the respective indication or application, or may be any scFv, Fab and/or fragment of the additional polypeptide as defined in **Table 4.**

**Table 4**

| **Indication / Application** | **Additional polypeptide** |
|---|---|
| Cytokines | IL-1β, IL-2, IL-10R, TGF-β |
| Cytokine receptors | IL-1Rs, IL-2Rs (e.g., CD25, CD122, CD132), IL-10R, TGFBR1 and TGFBR2 |
| Chemokines | CXCLs, CCLs, XCLs, CX3CLs |
| Chemokine receptors | CXCRs, CCRs, XCRs, CX3CRs |
| Immune ligands | B7 (e.g., CD80, CD86, PD-L1, PD-L2, ICOSL, VISTA), TNFSF (e.g., OX40L, 4-1BBL, TNFα, TNFβ, TRAIL, BAFF, CD40L), Peptide-loaded, AID-associated HLAs (e.g., HLA-DQ2, HLA-DQ8), classical and non-classical MHC class I and II molecules (e.g., HLA-E, HLA-G), BTLA, CD100, FGL-1, TIGIT, Collagen, VEGF |
| Immune receptors | CD28, CTLA4, PD-1, ICOS, TNFRSF (e.g., OX40, 4-1BB, TNFR2, TNFRSF14, TRAILR, BAFFR, CD40), LILRs (e.g., LILRA1 to LILRA6, LILRB1 to LILRB5), KIRs, CD155 (PVR), CD112 (PVRL2), CD72, LAIR1, TIM-3, LAG-3, CD31, CD66, VEGFR |

Preferably, for the use in the mitigation and/or treatment of a metabolic disease, the additional polypeptide may be selected from the group as defined in **Table 5**, but not limited thereof, or may be any scFv, Fab and/or fragment of the additional polypeptide as defined in **Table 5**.

**Table 5**

| **Indication / Application** | **Additional polypeptide** |
|---|---|
| Anemia | EPO |
| Weight, type 2 diabetes management, metabolic-dysfunction associated fatty liver disease (MAFLD), Metabolic dysfunction-associated steatohepatitis (MASH) | GLP-1 |

Preferably, for the use in at method for preventing and/or treating an allergy, the additional polypeptide may be an allergen comprising cat allergens, such as Fel d 1, grass allergens, such as Phl p 1 and Phl p 5, tree allergens, such as Bet v 1, peanut allergens, such as Ara h 1, or bee venom allergens, such as Api m 1, but not limited thereof.

In one embodiment a pharmaceutical composition according to the present invention is provided for use in a method of mitigating immunogenicity and promoting tolerance induction against at least one biological therapeutic.

One aspect relates to a kit comprising an scFc-engager and/or at least one nucleic acid molecule and/or the at least one vector and/or the at least one self-amplifying biologic according to the present invention, optionally wherein the kit comprises further reagents, including at least one buffer or solution.

### Examples

### Example 1

The following implementation example describes a procedure for designing, generating, selecting, and testing scFc variants. In particular, it details the procedures for tolerogenic CD32B-selective scFc variants, i.e., variants with high affinity to the inhibitory FcγR CD32B but low affinity to activating FcyRs CD64, CD32A, CD16A, and CD16B. Foremost, the focus is on the ratio of affinities of CD32B to CD32A (including both CD32A H131 and CD32A R131). The embodiment concludes with the description of the design, generation, characterization, and in vitro as well as in vivo testing of an scFc-engager that comprises hMOG (1-125) or other autoantigens, and a CD32B-selective scFc.

Design of genetic constructs. The protein sequence of the human IgG2 heavy chain (IGHG2_HUMAN, Accession number: P01859, Positions 222 to 447 according to Eu numbering) is selected as the scaffold for designing respective tolerogenic scFcs. This wild type (unmutated) protein sequence is referred to as first Fc-domain (Fc-domain I) and second Fc-domain (Fc-domain II), respectively, and at this stage of the process, the sequences of Fc-domain I and Fc-domain II are identical, making the design symmetrical. Fc-domain I and Fc-domain II are linked through the intermediate linker, specifically a (G₄S)₁₀ Linker, to form a wildtype (unmutated) human IgG2-based scFc. This in silico-generated protein sequence is subsequently translated into a respective mammalian codon-optimized DNA sequence, with particular attention paid to the following aspects. The sequence identity between the two Fc protomers comprising an scFc (namely, Fc-domain I and Fc-domain II) does not exceed 85% and the sequence identity is not below 65%. The length of identical sequence stretches are limited to no more than 25 base pairs. The DNA sequence of the (G₄S)₁₀ linker is designed to contain a minimum of homopolymers and codon repeats.

Synthesis of genetic constructs and yeast library generation. The creation of a yeast library encoding scFc variants with random mutations at specific sites are undertaken using the Reverse Kunkel method, as exemplified for an antigen-binding fragment (Fab) in Lin, W.-C. et al., Fragment-Directed Random Mutagenesis by the Reverse Kunkel Method, ACS Synth. Biol. (2022), 11, 4, 1658-1668, whereas any alternative molecular cloning method known in the art could be used. The library is constructed to include up to 10⁹ variants, derived from a theoretically possible number of variants several magnitudes larger, as calculated by the permutation of sites of interest within the mutagenesis space. It targets a mutation rate of 4 to 6 mutations within the variable regions of the WT IgG2 scFc (Hinge + CH2 + CH3) as defined in SEQ ID NO: 6. Initially, single-stranded DNA (ssDNA) oligonucleotides are generated through chemical synthesis. These oligonucleotides cover the forward sequences associated with the constant regions of the scFc (i.e., those areas that should not undergo random mutation) and also include a reverse complementary sequence that spans the entire length of the insert, namely WT IgG2 scFc (Hinge + CH2 + CH3) (SEQ ID NO: 6). Should mutations be introduced in Region 1A, 1D, 2A, 2B, 2C and 2D as depicted in FIG. 5, oligonucleotides covering the forward sequences must span the positions (according to Eu numbering) 222 to 232, 239 to 326, 331 to 447 of Fc-domain I, as well as 222 to 233, 238 to 264, 271 to 295, 299 to 326, and 328 to 447 of Fc-domain II. All oligonucleotides, except for the first covering the forward sequences from position 222 to 232, are synthesized as 5' phosphorylated oligonucleotides. After the annealing of the oligonucleotides covering the forward sequences with the reverse complementary sequence that spans the entire length, error-prone gap filling and ligation is performed.

Yeast surface display. After the clearance of the parental strand (i.e., the non-mutated antisense strand) with an appropriate exonuclease, the generated mutated scFc variants will be amplified with primers containing appropriate overhangs to facilitate ligation into Aga1p-Aga2p system plasmids. Saccharomyces cerevisiae EBY100 will serve as the host strain for the subsequent yeast display campaigns. Quality control measures will include sequencing to verify correctness, estimation of library diversity with next-generation sequencing, and phenotypic/conformational characterization, such as binding to Protein A and/or conformation-specific antibodies. The display of poorly folded scFc variants will be minimized by utilizing defined cultivation conditions that result in increased intracellular protein degradation of misfolded proteins. Several iterative rounds of positive and negative selection, enrichments with biotinylated, soluble (i.e., truncated) CD32B, and activating FcyRs will be performed using magnetic beads-based cell sorting (MACS) and fluorescence-activated cell sorting (FACS), respectively. scFc variants that exhibit the desired binding profile and minimal cross-reactivity between the full range of FcyRs will be identified through sequencing of relevant single clones and the evaluation of binding of appropriately labeled, soluble (i.e., truncated) CD32B and activating FcγR.

Expression and characterization of soluble scFc variants. scFc variants exhibiting appropriate binding properties, as enriched through the previously described yeast display campaigns, will be expressed in suitable mammalian suspension cells, such as human embryo kidney (HEK) cells or Chinese hamster ovary (CHO) cells. The down-selection of promising scFc variants, both before and after subjecting them to accelerated stability testing conditions, will be based on their biophysical and binding properties. These properties will include, but are not limited to, expression level, yield after Protein A-based purification, monomer content as determined by size-exclusion chromatography (SEC), and thermostability as determined by differential scanning calorimetry (DSC). Furthermore, their affinity towards activating FcyRs, inhibitory FcyRs, Siglecs, Clecs, FcRn (at relevant pH values), C1q, and CD305 will be measured using surface plasmon resonance (SPR), biolayer interferometry (BLI), and/or appropriate immuno- or cell-based assays, as required. scFc variants will then be further down-selected based on the correct processing of the N- and C-termini and relevant post-translational modifications, with particular emphasis on glycosylation. In addition to, or instead of, down selection based on the testing of biophysical and binding properties, a mammalian cell display step will be performed with variants enriched through the yeast display campaign. This approach will allow us to enrich variants with glycan-dependent binding properties, taking into account the different glycosylation profiles of yeast and mammalian cells. The mammalian cell display will be based on a system where the scFc is fused to the transmembrane domain of human PDGFR or other suitable anchoring domains, as described by I.a. by Ho, M., & Pastan, I., Mammalian Cell Display for Antibody Engineering, Therapeutic Antibodies, Methods in Molecular Biology™, 525, 337-352. We will assess the glycan profile and the occupancy of the expressed and purified scFc variants using lectin-based Western blot (WB) and mass spectrometry (MS).

In vitro evaluation of scFc variants in the context of hMOG (1-125) scFc-engager. To evaluate the tolerogenic properties of the selected CD32B-selective scFc variants, the scFc-domain will be fused to hMOG (1-125), as encoded by SEQ ID NO: 15, to create an scFc-engager. This protein will then be expressed in suitable mammalian suspension cells, such as human embryo kidney (HEK) cells or Chinese hamster ovary (CHO) cells, and purified through affinity chromatography, such as Protein A affinity chromatography, Size Exclusion Chromatography (SEC) and Ion Exchange Chromatography (IEX). The hMOG (1-125) scFc-engager will undergo characterization by the same methods as the corresponding scFc variant. Furthermore, the identity and conformational integrity of the hMOG (1-125) domain will be confirmed through Western Blot (WB) using appropriate antibodies. To ensure sterility and the absence of endotoxin, appropriate assays will be performed. A series of reporter assay-based experiments will be devised to confirm the tolerogenic properties of the molecules. To study the potential effect on dendritic cells, a Jurkat cell-based reporter assay will be utilized. The Jurkat cell will be transduced to express both signalling-competent, full-size CD32A and CD32B. Signalling through the activating FcγR CD32A, achieved through crosslinking by immunoactivating, pathogenic immune complexes (i.e., complexes of autoantigen with respective autoantibodies; in this case, hMOG (1-125) and anti-hMOG (1-125) antibodies), will trigger a cascade through NF-κB that results in the expression of green fluorescent protein (GFP), which can be monitored by various methods such as FACS. This signal will be inhibited in a dose-dependent manner by simultaneous signalling through the inhibitory FcγR CD32B. To evaluate the tolerogenic properties of hMOG (1-125) scFc-engager, these scFc-engagers will be mixed with respective immune complexes to form hybrid immune complexes with altered immunomodulating properties. Those hMOG (1-125) scFc-engagers with the lowest half-maximal effective concentration (EC50) will be selected for further studies. Similarly, to assess the potential effect on autoreactive B cells, a Jurkat cell-based reporter assay will be employed. Here, instead of activating FcγR CD32A, the autoreactive BCR along with its B-cell antigen receptor complex-associated proteins (i.e., CD79A and CD79B) will be used to trigger the NF-κB cascade. To explore the impact of other inhibitory receptors, Jurkat cells will be transduced with respective signalling-competent receptors such as CD22 (Siglec-2), CD23 (Fc epsilon RII or FcεRII), CD33 (Siglec-3), CD328 (Siglec-7), CD329 (Siglec-9), and Siglec-10.

Ex vivo evaluation of scFc variants in the context of hMOG (1-125) scFc-engager. To confirm the tolerogenic properties of the hMOG (1-125) scFc-engager in a potentially more relevant primary human dendritic cell system, appropriate experiments involving human monocyte-derived dendritic cells (moDCs) will be devised. Analogous to Jurkat cell-based reporter assays, immunoactivating, pathogenic immune complexes will be used to evaluate the tolerogenic properties of hMOG (1-125) scFc-engager. moDCs and PBMCs will be isolated from buffy coats using commonly used density gradient media. CD14+ monocytes will then be further isolated by positive selection using a magnetic activated cell sorting (MACS) method. The differentiation of monocytes to moDCs will occur by cultivating them in the presence of IL-4 and GM-CSF. After 6 days, fully differentiated moDCs will be activated by immune complexes or immune complexes + hMOG (1-125) scFc-engagers. The scFc-engagers within ICs are expected to induce a tolerogenic DC (tolDC) state characterized by a low ratio of co-stimulatory to co-inhibitory receptor expression (costimulatory < coinhibitory) and the production of anti-inflammatory cytokines such as IL-10 and TGFβ.

In vivo evaluation of scFc variants in the context of hMOG (1-125) scFc-engager. To confirm the tolerogenic properties of the hMOG (1-125) scFc-engager in vivo, the experimental autoimmune encephalomyelitis (EAE) model will be utilized in two different setups, a prophylactic and a therapeutic one. FcγR humanized mice will be immunized with hMOG (1-125)/complete Freund's adjuvant (CFA) subcutaneously and pertussis toxin intraperitoneally on day 0. The animals will then be intravenously injected with the hMOG (1-125) scFc-engager. For the prophylactic setup, animals will be treated from day 5 to 7, i.e., before the disease onset, as assessed by the clinical score. For the therapeutic setup, animals will be treated from day 12 onwards, i.e., after the mice have developed symptoms through the treatment with hMOG (1-125)/ complete Freund's adjuvant (CFA) and pertussis toxin. The evaluation of the tolerogenic properties will involve detailed analysis of both T and B cells. This will encompass a range of cellular responses, including: the assessment of naive cells, effector and effector memory cells; the measurement of T cell proliferation; the examination of the regulatory T (Treg) cell population; and the analysis of MOG-specific T cells, which will be determined by their cytokine production in response to restimulation with a MOG peptide pool. Additionally, the study will quantify the percentage of MOG-specific B cells and evaluate the humoral (antibody-mediated) anti-MOG response. Following treatment with the scFc-engager, the expectation is that MOG-specific T and B cell responses will be significantly reduced or completely stopped. This suppression of immune responses is anticipated to lead to a positive outcome in the disease condition.

### Example 2

The following example describes the procedure for designing, generating, selecting, and testing scFc. In particular, it details the procedures for immunogenic activating FcγR-selective scFc variants, i.e., variants with low affinity to the inhibitory FcγR CD32B but high affinity to activating FcyRs CD64, CD32A, CD16A, and CD16B. Foremost, the focus is on the ratio of affinities of CD32B to CD32A (both CD32A H131 and CD32A R131). The embodiment concludes with the description of the design, generation, characterization, and in vitro as well as in vivo testing of a scFc-engager that comprises OVA (1-386) or other immunogens and a CD32A-selective scFc.

Design of genetic constructs. The protein sequence of the human IgG1 heavy chain (IGHG1_HUMAN, Accession number: P01857, Positions 221 to 447 according to Eu numbering) is selected as the scaffold for designing respective immunogenic scFcs. This wild type (unmutated) protein sequence is referred to as Fc-domain I and Fc-domain II, respectively, and at this stage of the process, the sequences of Fc-domain I and Fc-domain II are identical, making the design symmetrical. Fc-domain I and Fc-domain II are linked through the intermediate linker, specifically a (G₄S)₁₀ Linker, to form a wildtype (unmutated) human IgG1-based scFc. This in silico-generated protein sequence is then translated into a respective mammalian codon-optimized DNA sequence, with particular attention paid to the following aspects. The sequence identity between the two Fc protomers comprising an scFc (namely, Fc-domain I and Fc-domain II) does not exceed 85% and the sequence identity is below 65%. The length of identical sequence stretches are limited to no more than 25 base pairs. The DNA sequence of the (G₄S)₁₀ linker is designed to contain a minimum of homopolymers and codon repeats.

Synthesis of genetic constructs and yeast library generation. The creation of a yeast library encoding scFc variants with random mutations at specific sites is undertaken using error-prone PCR-based (epPCR-based) approaches, although numerous alternative methods are available. The library is constructed to include up to 10^9 variants, derived from a theoretically possible number of variants several magnitudes larger, as calculated by the permutation of sites of interest within the mutagenesis space. It targets a mutation rate of 4 to 6 mutations within the variable regions of the WT IgG1 scFc (truncated hinge + CH2 + CH3) as defined in SEQ ID NO 1. Mutations are introduced in Region 1A, 1D, 2A, 2B, 2C, and 2D, as indicated in FIG. 5. Double-stranded DNA (dsDNA) templates, encoding these regions along with stretches of appropriate length (i.e., 50 to 100 bp), complementary to the scFc regions upstream and downstream of these, are synthesized. For example, Region 1A, which covers the amino acid positions 232 to 243, will necessitate the synthesis of double-stranded DNA extending from position 207 to 268. Primers for epPCR are designed to flank the regions selected for mutation and anneal to the entire length of the stretches upstream and downstream of these regions. The introduction of mutations is realized through the use of lower fidelity polymerases, manipulation of MgCl₂ concentration, unbalanced dNTP mixtures, or the use of nucleotide analogs, as appropriate. Subsequently, dsDNA templates of the unmutated regions of the WT IgG1 scFc (i.e., all those regions in which no mutations should be introduced) along with stretches of appropriate length (i.e., 50 to 100 bp) are synthesized. These stretches are complementary to the unmutated stretches of the DNA fragments that underwent the epPCR. These PCR products and templates are then introduced into yeast cells along with an appropriate Aga1p-Aga2p plasmid backbone under conditions that will enable homologous recombination, resulting in a library of randomly mutated and homologously recombined scFc variants.

Yeast surface display. Saccharomyces cerevisiae EBY100 will serve as the host strain for the subsequent yeast display campaigns. Quality control measures will include sequencing to verify correctness, estimation of library diversity with next-generation sequencing, and phenotypic/conformational characterization, such as binding to Protein A and/or conformation-specific antibodies. The display of poorly folded scFc variants will be minimized by utilizing defined cultivation conditions that result in increased intracellular protein degradation of misfolded proteins. Several iterative rounds of positive and negative selection, enrichments with biotinylated, soluble (i.e., truncated) CD32B, and activating FcyRs will be performed using magnetic beads-based cell sorting (MACS) and fluorescence-activated cell sorting (FACS), respectively. scFc variants with the desired binding profile and minimal cross-reactivity between the full range of FcyRs will be identified through sequencing of relevant single clones and the evaluation of binding of appropriately labeled, soluble (i.e., truncated) CD64, CD32A, CD16A, CD16B, and the inhibiting FcγR CD32B.

Expression and characterization of soluble scFc variants. scFc variants exhibiting appropriate binding properties, as enriched through the previously described yeast display campaigns, will be expressed in suitable mammalian suspension cells, such as human embryo kidney (HEK) cells or Chinese hamster ovary (CHO) cells. The down-selection of promising scFc variants, both before and after subjecting them to accelerated stability testing conditions, will be based on their biophysical and binding properties. These properties will include, but are not limited to, expression level, yield after Protein A-based purification, monomer content as determined by size-exclusion chromatography (SEC), and thermostability as determined by differential scanning calorimetry (DSC). Furthermore, their affinity towards activating FcyRs, inhibitory FcyRs, Siglecs, Clecs, FcRn (at relevant pH values), C1q, and CD305 will be measured using surface plasmon resonance (SPR), biolayer interferometry (BLI), and/or appropriate immuno- or cell-based assays, as required. scFc variants will then be further down-selected based on the correct processing of the N- and C-termini and relevant post-translational modifications, with particular emphasis on glycosylation. In addition to, or instead of, downselection based on the testing of biophysical and binding properties, a mammalian cell display step will be performed with variants enriched through the yeast display campaign. This approach will allow us to enrich variants with glycan-dependent binding properties, taking into account the different glycosylation profiles of yeast and mammalian cells. The mammalian cell display will be based on a system where the scFc is fused to the transmembrane domain of human PDGFR or other suitable anchoring domains, as described by I.a. by Ho, M., & Pastan, I., Mammalian Cell Display for Antibody Engineering, Therapeutic Antibodies, Methods in Molecular Biology™, 525, 337-352. We will assess the glycan profile and the occupancy of the expressed and purified scFc variants using lectin-based Western blot (WB) and mass spectrometry (MS).

In vitro evaluation of scFc variants in the context of OVA (1-386) scFc-engager. To evaluate the immunogenic properties of the selected activating FcγR -selective scFc variants, the scFc-domain will be fused to OVA (1-386), as encoded by SEQ ID NO: 16, to create an scFc-engager. This protein will then be expressed in suitable mammalian suspension cells, such as human embryo kidney (HEK) cells or Chinese hamster ovary (CHO) cells, and purified through affinity chromatography, such as Protein A affinity chromatography, Size Exclusion Chromatography (SEC) and Ion Exchange Chromatography (IEX). The OVA (1-386) scFc-engager will undergo characterization by the same methods as the corresponding scFc variant. Furthermore, the identity and conformational integrity of the OVA (1-386) domain will be confirmed through Western Blot (WB) using appropriate antibodies. To ensure sterility and the absence of endotoxin, appropriate assays will be performed. A series of reporter assay-based experiments will be devised to confirm the immunogenic properties of the molecules. To study the potential effect on dendritic cells, a Jurkat cell-based reporter assay will be utilized. The Jurkat cell will be transduced to express both signalling-competent, full-size CD32A and CD32B. Signalling through the activating FcγR CD32A, achieved through crosslinking by immunoactivating, pathogenic immune complexes (i.e., complexes of autoantigen with respective autoantibodies; in this case, OVA (1-386) and anti-OVA (1-386) antibodies), will trigger a cascade through NF-κB that results in the expression of green fluorescent protein (GFP), which can be monitored by various methods such as FACS. This signal will be inhibited in a dose-dependent manner by simultaneous signalling through the inhibitory FcγR CD32B. This will be achieved by the addition of CD32B-selective anti-OVA (1-386) antibodies. To evaluate the immunogenic properties of OVA (1-386) scFc-engager, these scFc-engagers will be mixed with respective immune complexes and the CD32B-selective anti-OVA (1-386) antibodies to form hybrid immune complexes with altered immunomodulating properties. For further studies, those OVA (1-386) scFc-engagers that will achieve the lowest half-maximal effective concentration (EC50), indicating high potency, even in the presence of inhibitory CD32B-selective anti-OVA (1-386) antibodies, will be selected. Similarly, to assess the potential effect on autoreactive B cells, a Jurkat cell-based reporter assay will be employed. Here, instead of activating FcγR CD32A, the autoreactive BCR along with its B-cell antigen receptor complex-associated proteins (i.e., CD79A and CD79B) will be used to trigger the NF-κB cascade. To explore the impact of other inhibitory receptors, Jurkat cells will be transduced with respective signalling-competent receptors such as CD22 (Siglec-2), CD23 (Fc epsilon RII or FcεRII), CD33 (Siglec-3), CD328 (Siglec-7), CD329 (Siglec-9), and Siglec-10.

Ex vivo evaluation of scFc variants in the context of OVA (1-386) scFc-engager. To confirm the immunogenic properties of the OVA (1-386) scFc-engager in a potentially more relevant primary human dendritic cell system, appropriate experiments involving human monocyte-derived dendritic cells (moDCs) will be devised. Analogous to Jurkat cell-based reporter assays, immunoactivating, pathogenic immune complexes will be used to evaluate the immunogenic properties of OVA (1-386) scFc-engager. moDCs and PBMCs will be isolated from buffy coats using commonly used density gradient media. CD14+ monocytes will then be further isolated by positive selection using a magnetic activated cell sorting (MACS) method. The differentiation of monocytes to moDCs will occur by cultivating them in the presence of IL-4 and GM-CSF. After 6 days, fully differentiated moDCs will be activated by immune complexes or immune complexes + OVA (1-386) scFc-engagers or immune complexes + OVA (1-386) scFc-engagers + CD32B-selective anti- OVA (1-386) antibodies. The scFc-engagers within immune complexes (ICs) are expected to induce a mature dendritic cell (mDC) state characterized by a high ratio of co-stimulatory to co-inhibitory receptor expression (costimulatory > coinhibitory) and the production of pro-inflammatory cytokines such as IL-12 and TNFα.

In vivo evaluation of scFc variants in the context of OVA (1-386) scfc-engager. To evaluate the immunogenic properties of the OVA (1-386) scFc-engagers in vivo, an OVA immunization model will be employed. On day 0, mice will undergo subcutaneous immunization with OVA/complete Freund's adjuvant (CFA). Following this initial immunization step, on day 13, CFSE-labelled OVA-specific CD4 (OT-II) and CD8 (OT-I) T cells will be intravenously transferred into the mice. This procedure is designed to monitor the activation and proliferation of these antigen-specific T cells accurately. A booster immunization with OVA/incomplete Freund's adjuvant (IFA) or OVA (1-386) scFc-engagers / incomplete Freund's adjuvant (IFA) or appropriate controls (e.g., an immunologically inert OVA (1-386) scFc-engager) will be given subcutaneously on day 14. On day 21 to day 35, spleen and inguinal lymph nodes will be harvested to undergo comprehensive immunological assessments. T and B cell populations will be characterized, including naive, effector, effector memory, and OVA-specific T cells based on cytokine production following restimulation with an OVA peptide pool. The proliferation of transferred T cells will be measured by CFSE dilution. The analysis will also determine the fate of these T cells, including the percentage of regulatory T cells (Tregs) and cytokine production, and quantify the percentage of OVA-specific B cells, particularly those in germinal centers. The OVA (1-386) scFc-engager will be expected to exhibit improved anti-OVA immune responses in comparison to both the standard OVA immunization and the control treatments.

### Example 3

The following example describes the procedure for designing, generating, selecting, and testing specific scFc variants. In particular, it details the procedures for immunologically inert scFc variants, i.e., variants with no binding to any FcyRs. Foremost, the focus is on maintaining binding to both Protein A and FcRn while abolishing binding to all FcyRs. The embodiment concludes with the description of the design, generation, characterization, and in vitro as well as in vivo testing of an scFc-engager that comprises IL-10 or other immunomodulatory proteins and an immunologically inert scFc.

Design of genetic constructs. The protein sequence of the human IgG2 heavy chain (IGHG2_HUMAN, Accession number: P01859, Positions 222 to 447 according to Eu numbering) is selected as the scaffold for designing respective immunologically inert scFcs. This wild type (unmutated) protein sequence is referred to as Fc-domain I and Fc-domain II, respectively, and at this stage of the process, the sequences of Fc-domain I and Fc-domain II are identical, making the design symmetrical. Fc-domain I and Fc-domain II are linked through the intermediate linker, specifically a (G₄S)₁₀ Linker, to form a wildtype (unmutated) human IgG2-based scFc. This in silico-generated protein sequence is then translated into a respective mammalian codon-optimized DNA sequence, with particular attention paid to the following aspects. The sequence identity between the two Fc protomers comprising an scFc (namely, Fc-domain I and Fc-domain II) does not exceed 85%; the sequence identity is below 65%. The length of identical sequence stretches is limited to no more than 25 base pairs. The DNA sequence of the (G₄S)₁₀ linker is designed to contain a minimum of homopolymers and codon repeats.

Synthesis of genetic constructs and yeast library generation. The creation of a yeast library encoding scFc variants with random mutations at specific sites is undertaken using degenerated primer PCR-based approaches, whereas numerous alternative methods are available. The library is constructed to include up to 10^9 variants, derived from a theoretically possible number of variants several magnitudes larger, as calculated by the permutation of sites of interest within the mutagenesis space. It targets a mutation rate of 4 to 6 mutations within the relevant regions of the WT IgG2 scFc (Hinge + CH2 + CH3) as defined in SEQ ID NO: 6). Mutations will be introduced in Region 1A, 1D, 2A, 2B, 2C and 2D as depicted in FIG. 5. Double-stranded DNA (dsDNA) templates, encoding these regions along with stretches of appropriate length (i.e., 25 to 50 bp), complementary to the scFc regions upstream and downstream of these, are synthesized. For example, Region 1A, which covers the amino acid positions 232 to 243, necessitates the synthesis of double-stranded DNA extending from position 222 to 256. Degenerated primer (e.g., with NNK that encode all 20 amino acids or other suitable codons) is appropriately designed to specify the region to be mutated as well as to specify the region to be left unmutated and at which annealing occurs efficiently through the use of complementary sequences. The latter facilitates efficient homologous recombination in subsequent steps of the library generation. Subsequently, dsDNA templates of the unmutated regions of the WT IgG2 scFc (i.e., all those regions in which no mutations should be introduced) along with stretches of appropriate length (i.e., 50 to 100 bp) are synthesized. These stretches are complementary to the unmutated stretches of the DNA fragments that underwent the degenerated primer PCR. These PCR products and templates will then be introduced into yeast cells along with an appropriate Aga1p-Aga2p plasmid backbone under conditions that enable homologous recombination, resulting in a library of randomly mutated and homologously recombined scFc variants.

Yeast surface display. Saccharomyces cerevisiae EBY100 will serve as the host strain for the subsequent yeast display campaigns. Quality control measures will include sequencing to verify correctness, estimation of library diversity with next-generation sequencing, and phenotypic/conformational characterization, such as binding to Protein A and/or conformation-specific antibodies. The display of poorly folded scFc variants will be minimized by utilizing defined cultivation conditions that result in increased intracellular protein degradation of misfolded proteins. Several iterative rounds of positive and negative selection, enrichments with biotinylated, soluble ((i.e., truncated) FcyRs and Protein A will be performed using magnetic beads-based cell sorting (MACS) and fluorescence-activated cell sorting (FACS), respectively. scFc variants with the desired binding profile will be identified through sequencing of relevant single clones and the evaluation of binding of appropriately labeled, soluble (i.e., truncated) FcyRs and Protein A.

Expression and characterization of soluble scFc variants. scFc variants with appropriate binding properties as enriched through the above-described yeast display campaigns are expressed in appropriate mammalian suspension cells, e.g., human embryo kidney (HEK) cells or Chinese hamster ovary (CHO) cells. The down selection of promising scFc variants (before and after subjecting them accelerated stability testing conditions) is performed based on their biophysical and binding properties. These properties include (but are not limited to) expression level, yield after Protein A-based purification, monomer content as determined by size-exclusion chromatography (SEC), thermostability as determined by differential scanning calorimetry (DSC). Moreover, affinity towards activating FcyRs, inhibitory FcyRs, Siglecs, Clecs, FcRn (at relevant pH values), C1q and CD305 are measured with surface plasmon resonance (SPR), biolayer interferometry (BLI) and/or appropriate immuno- or cell-based assays, as required. scFc variants are then further down selected on the basis of correct processing of the N- and C-terminus and relevant post-translational modification with particular emphasis on glycosylation. In addition to, or instead of, down selection based on the testing of biophysical and binding properties, a mammalian cell display step will be performed with variants enriched through the yeast display campaign. This approach will allow us to enrich variants with glycan-dependent binding properties, taking into account the different glycosylation profiles of yeast and mammalian cells. The mammalian cell display will be based on a system where the scFc is fused to the transmembrane domain of human PDGFR or other suitable anchoring domains, as described by I.a. by Ho, M., & Pastan, I., Mammalian Cell Display for Antibody Engineering, Therapeutic Antibodies, Methods in Molecular Biology™, 525, 337-352. We will assess the glycan profile and the occupancy of the expressed and purified scFc variants using lectin-based Western blot (WB) and mass spectrometry (MS).

In vitro evaluation of IL-10 scfc-engager. Single chain IL-10 (sclL-10), as encoded by SEQ ID NO: 17, and scIL-10 scFc-engager will be tested side by side to investigate whether attaching scIL-10 to the immunologically inert scFc alters its ability to bind to the IL-10R: This will involve comparative analysis using the commercially available HEK-Blue^{™} IL-10 cells, which are specifically designed to detect bioactive human IL-10 by monitoring the activation of the STAT3 pathway.

## Claims

1. An scFc-engager comprising:
(i) a first Fc-domain polypeptide comprising or consisting of a hinge-CH2-CH3 domain,
(ii) a second Fc-domain polypeptide comprising or consisting of a hinge-CH2-CH3 domain,
(iii) optionally: at least one, additional polypeptide comprising at least one immunomodulatory domain and/or at least one targeting domain, wherein the at least one additional polypeptide is positioned at N-terminally and/or the C-terminally at the first and/or the second Fc-domain polypeptide;
(iv) and optionally wherein the at least one additional polypeptide and the first and/or second Fc-domain polypeptide respectively, are connected by a linker.
(v) an interdomain linker connecting the first Fc-domain polypeptide of (i) with the second Fc-domain polypeptide of (ii), and
(vi) optionally: a multimerization domain between the at least one additional polypeptide of (iii) and the first Fc-domain polypeptide of (i), and/or optionally, a multimerization domain fused to the C-terminus of the second Fc-domain,
wherein the amino acid sequence of the first Fc-domain polypeptide and the amino acid sequence of the second Fc-domain polypeptide differ by at least one functional mutation, wherein the at least one functional mutation is configured to modulate the binding affinity between the combined first and second Fc-domain polypeptide and an immune receptor.

2. The scFc-engager according to claim 1, wherein the at least one additional polypeptide of (iii) comprises or consists of at least one of an autoantigen, pathogen-associated antigen, cytokine and/or cytokine receptor, immune checkpoint ligand and/or at least one receptor, tumor/cancer-associated (neo)antigen, artificial epitope, imaging/reporter protein, biomarker, enzyme, antibody or variant, or fragment thereof; and optionally at least one immunomodulatory domain and/or at least one targeting domain, or any combination thereof.

3. The scFc-engager according to claim 1 or 2, wherein the linker of the at least one additional polypeptide and/or the interdomain linker comprise(s) at least one glycan site, preferably a NXT/S motif, and/or at least one stabilizing amino acid mutation and/or at least one binding affinity modulating mutation.

4. The scFc-engager according to any of the preceding claims, wherein the first or second Fc-engager domain polypeptide has an amino acid sequence as defined by SEQ ID NOs: 1 to 8, or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any one of SEQ ID NOs: 1 to 8.

5. At least one nucleic acid molecule encoding the scFc-engager according to any one of the preceding claims.

6. At least one vector comprising at least one nucleic acid molecule as defined in claim 5, encoding the scFc-engager according to any one of claims 1 to 4.

7. At least one self-amplifying biologic, comprising the at least one nucleic acid molecule of claim 5 or the at least one vector of claim 6.

8. A method of producing the scFc-engager according to any one of claims 1 to 4, the method comprising:
(i) introducing the at least one nucleic acid molecule of claim 5, the at least one vector of claim 6, or the at least one self-amplifying biologic of claim 7 into a host cell or in vitro translation system,
(ii) culturing or incubating the at least one host cell or the at least one in vitro translation system under conditions suitable for transcription and/or translation of the at least one nucleic acid molecule or the at least one vector,
(iii) allowing the assembly of the scFc-engager or the at least one self-amplifying biologic encoded by the at least one nucleic acid molecule or the at least one vector; and
(iv) optionally: purifying the scFc-engager or the self-amplifying biologic of step (iii); and
(v) obtaining the scFc-engager or the at least one self-amplifying biologic.

9. The method of claim 8 further comprising a step of:
(vi) introducing or contacting the scFc-engager or the at least one self-amplifying biologic, as obtained in step (v) of claim 8 into/with a target cell or into/with an in vitro cellular system for transfecting said at least one target cell and/or for studying the functionality of the scFc-engager or the at least one self-amplifying biologic.

10. In vitro use of the scFc-engager according to claims 1 to 4, wherein the scFc-engager is brought into contact with a biological sample.

11. A pharmaceutical composition comprising the scFc-engager according to claims 1 to 4, or a pharmaceutical composition comprising the scFc-engager obtained by or obtainable by a method according to claim 8, and optionally, at least one pharmaceutically acceptable carrier or excipient.

12. A pharmaceutical composition comprising the at least one self-amplifying biologic as according to claim 7, or a pharmaceutical composition comprising the at least one self-amplifying biologic obtained by or obtainable by a method according to claim 8, and optionally, at least one pharmaceutically acceptable carrier or excipient.

13. A pharmaceutical composition according to claims 11 and 12, for use in a method of treating and/or preventing a disease, wherein the disease is independently selected from at least one immune disease, autoimmune disease or autoimmune-like condition, at least one inflammatory disease, such as an allergen-, bacterial or viral associated inflammation, a cardiovascular disease and at least one cancer.

14. A pharmaceutical composition according to claims 11 and 12 for use in a method of mitigating immunogenicity and/or promoting tolerance induction against at least one biological therapeutic.

15. A kit comprising the scFc-engager according to claims 1 to 4 and/or the at least one nucleic acid molecule according to claim 5 and/or the at least one vector according to claim 6 and/or the at least one self-amplifying biologic according to claim 7, optionally wherein the kit comprises further reagents, including at least one buffer or solution.
